# EUROPEAN PATENT SPECIFICATION

(11) **EP 2 038 074 B1**
(45) Date of publication and mention of the grant of the patent: **11.06.2014**
(21) Application number: 07812529.1
(22) Date of filing: 29.06.2007
(51) Int. Cl.: B01J 19/00, B82Y 30/00, C40B 50/14, G01N 33/68, C40B 40/04, G01N 33/543

(54) **MAKE AND USE OF SURFACE MOLECULES OF VARIED DENSITIES**
HERSTELLUNG UND VERWENDUNG VON OBERFLÄCHENMOLEKÜLEN VON UNTERSCHIEDLICHER DICHTE
FABRICATION ET UTILISATION DE MOLÉCULES DE SURFACE À DIFFÉRENTES DENSITÉS

(30) Priority: 29.06.2006 US 817498 P
(43) Date of publication of application: 25.03.2009
(73) Proprietor: Gao, Xiaolian, Houston, TX 77030 (US); Zhou, Xiaochuan, Houston, TX 77030 (US); Zhang, Xiaolin, Sugar Land TX 77479 (US); Hong, Ailing, Bellaire TX 77401 (US); Zhu, Qi, Houston TX 77025 (US); Yu, Peilin, Houston TX 77081 (US)
(72) Inventor: Gao, Xiaolian, Houston, TX 77030 (US); Zhou, Xiaochuan, Houston, TX 77030 (US); Zhang, Xiaolin, Sugar Land TX 77479 (US); Hong, Ailing, Bellaire TX 77401 (US); Zhu, Qi, Houston TX 77025 (US); Yu, Peilin, Houston TX 77081 (US)
(74) Representative: Longland, Emma Louise
(86) International application number: PCT/US2007/072609
(87) International publication number: WO 2008/003100

(56) References cited:
- EP-A- 0 895 082
- EP-A- 1 081 163
- EP-A- 1 645 639
- US-A- 5 744 305
- US-A1- 2002 006 622
- US-A1- 2002 039 728
- US-A1- 2003 198 943
- US-A1- 2004 092 396
- US-B1- 6 184 347
- XIAOLIAN GAO ET AL: "High density peptide microarrays. In situ synthesis and applications" MOLECULAR DIVERSITY, KLUWER ACADEMIC PUBLISHERS, DO, vol. 8, no. 3, 1 September 2004 (2004-09-01), pages 177-187, XP019258545 ISSN: 1573-501X
- GAO X ET AL: "In situ synthesis of oligonucleotide microarrays" BIOPOLYMERS, NEW YORK, NY, US, vol. 73, no. 5, 5 April 2004 (2004-04-05), pages 579-596, XP002346385 ISSN: 0006-3525
- GAO X. ET AL.: 'High density peptide microarrays. In situ synthesis and applications' MOLECULAR DIVERSITY vol. 8, no. 3, 2004, pages 177 - 187, XP019258545
- GAO ET AL.: 'In Situ Synthesis of Oligonucleotide Microarrays' BIOPOLYMERS vol. 73, no. 5, 2004, pages 579 - 596, XP002346656

### SEQUENCE LISTING

<110> Gao, Xiaolian
<120> MAKE AND USE OF SURFACE MOLECULES OF VARIED DENSITIES
<130> Xiaolian Gao
<150> 60/817498
   <151> 2006-06-29
<160> 126
<170> PatentIn version 3.4
<210> 1
   <211> 4
   <212> PRT
   <213> Artificial
<220>
   <223> Chemical Synthesized
<400> 1
   Asp Tyr Lys His
   1
<210> 2
   <211> 4
   <212> PRT
   <213> Artificial
<220>
   <223> Chemical Synthesized
<400> 2
   Asp Tyr Lys Ala
   1
<210> 3
   <211> 4
   <212> PRT
   <213> Artificial
<220>
   <223> Chemical Synthesized
<400> 3
   Asp Tyr Lys Trp
   1
<210> 4
   <211> 9
   <212> PRT
   <213> Artificial
<220>
   <223> Chemical Synthesized
<400> 4
   Tyr Pro Tyr Asp Val Pro Asp Tyr Ala
   1 5
<210> 5
   <211> 4
   <212> PRT
   <213> Artificial
<220>
   <223> Chemical Synthesized
<400> 5
   Tyr Val Pro Met
   1
<210> 6
   <211> 4
   <212> PRT
   <213> Artificial
<220>
   <223> Chemical Synthesized
<400> 6
   Ala Val Pro Met
   1
<210> 7
   <211> 4
   <212> PRT
   <213> Artificial
<220>
   <223> Chemical synthsized
<220>
   <221> misc_feature
   <222> (1)..(1)
   <223> Xaa=phosphoTyrosine
<400> 7
   Xaa Val Pro Met
   1
<210> 8
   <211> 4
   <212> PRT
   <213> Artificial
<220>
   <223> Chemical Synthesized
<400> 8
   Tyr Glu Glu Ile
   1
<210> 9
   <211> 4
   <212> PRT
   <213> Artificial
<220>
   <223> Chemical Synthesized
<400> 9
   Ala Glu Glu Ile
   1
<210> 10
   <211> 4
   <212> PRT
   <213> Artificial
<220>
   <223> Chemical synthesized
<220>
   <221> misc_feature
   <222> (1)..(1)
   <223> Xaa=phosphoTyrosine
<400> 10
   Xaa Glu Glu Ile
   1
<210> 11
   <211> 5
   <212> PRT
   <213> Artificial
<220>
   <223> Chemical synthesized
<400> 11
   Tyr Glu Glu Ile Pro
   1 5
<210> 12
   <211> 5
   <212> PRT
   <213> Artificial
<220>
   <223> Chemical synthesized
<400> 12
   Tyr Glu Glu Ile Pro
   1 5
<210> 13
   <211> 5
   <212> PRT
   <213> Artificial
<220>
   <223> Chemical synthesized
<400> 13
   Ala Glu Glu Ile Pro
   1 5
<210> 14
   <211> 5
   <212> PRT
   <213> Artificial
<220>
   <223> Chemical synthesized
<220>
   <221> misc_feature
   <222> (1)..(1)
   <223> Xaa=phosphoTyrosine
<400> 14
   Xaa Glu Glu Ile Pro
   1 5
<210> 15
   <211> 5
   <212> PRT
   <213> Artificial
<220>
   <223> Chemical synthesized
<400> 15
   Arg Arg Ala Ser Leu
   1 5
<210> 16
   <211> 5
   <212> PRT
   <213> Artificial
<220>
   <223> Chemical synthesized
<400> 16
   Arg Arg Cys Ser Leu
   1 5
<210> 17
   <211> 5
   <212> PRT
   <213> Artificial
<220>
   <223> Chemical synthesized
<400> 17
   Arg Arg Asp Ser Leu
   1 5
<210> 18
   <211> 5
   <212> PRT
   <213> Artificial
<220>
   <223> Chemical synthesized
<400> 18
   Arg Arg Glu Ser Leu
   1 5
<210> 19
   <211> 5
   <212> PRT
   <213> Artificial
<220>
   <223> Chemical synthesized
<400> 19
   Arg Arg Phe Ser Leu
   1 5
<210> 20
   <211> 5
   <212> PRT
   <213> Artificial
<220>
   <223> Chemical synthesized
<400> 20
   Arg Arg Gly Ser Leu
   1 5
<210> 21
   <211> 5
   <212> PRT
   <213> Artificial
<220>
   <223> Chemical synthesized
<400> 21
   Arg Arg His Ser Leu
   1 5
<210> 22
   <211> 5
   <212> PRT
   <213> Artificial
<220>
   <223> Chemical synthesized
<400> 22
   Arg Arg Ile Ser Leu
   1               5
<210> 23
   <211> 5
   <212> PRT
   <213> Artificial
<220>
   <223> Chemical synthesized
<400> 23
   Arg Arg Lys Ser Leu
   1               5
<210> 24
   <211> 5
   <212> PRT
   <213> Artificial
<220>
   <223> Chemical synthesized
<400> 24
   Arg Arg Leu Ser Leu
   1               5
<210> 25
   <211> 5
   <212> PRT
   <213> Artificial
<220>
   <223> Chemical synthesized
<400> 25
   Arg Arg Met Ser Leu
   1               5
<210> 26
   <211> 5
   <212> PRT
   <213> Artificial
<220>
   <223> Chemical synthesized
<400> 26
   Arg Arg Asn Ser Leu
   1 5
<210> 27
   <211> 5
   <212> PRT
   <213> Artificial
<220>
   <223> Chemical synthesized
<400> 27
   Arg Arg Arg Ser Leu
   1 5
<210> 28
   <211> 5
   <212> PRT
   <213> Artificial
<220>
   <223> Chemical synthesized
<400> 28
   Arg Arg Pro Ser Leu
   1 5
<210> 29
   <211> 5
   <212> PRT
   <213> Artificial
<220>
   <223> Chemical synthesized
<400> 29
   Arg Arg Gln Ser Leu
   1 5
<210> 30
   <211> 5
   <212> PRT
   <213> Artificial
<220>
   <223> Chemical synthesized
<400> 30
   Arg Arg Ala Ala Leu
   1 5
<210> 31
   <211> 5
   <212> PRT
   <213> Artificial
<220>
   <223> Chemical synthesized
<400> 31
   Arg Arg Cys Ala Leu
   1               5
<210> 32
   <211> 5
   <212> PRT
   <213> Artificial
<220>
   <223> Chemical synthesized
<400> 32
   Arg Arg Asp Ala Leu
   1               5
<210> 33
   <211> 5
   <212> PRT
   <213> Artificial
<220>
   <223> Chemical synthesized
<400> 33
   Arg Arg Glu Ala Leu
   1               5
<210> 34
   <211> 5
   <212> PRT
   <213> Artificial
<220>
   <223> Chemical synthesized
<400> 34
   Arg Arg Phe Ala Leu
   1               5
<210> 35
   <211> 5
   <212> PRT
   <213> Artificial
<220>
   <223> Chemical synthesized
<400> 35
   Arg Arg Gly Ala Leu
   1               5
<210> 36
   <211> 5
   <212> PRT
   <213> Artificial
<220>
   <223> Chemical synthesized
<400> 36
   Arg Arg His Ala Leu
   1               5
<210> 37
   <211> 5
   <212> PRT
   <213> Artificial
<220>
   <223> Chemical synthesized
<400> 37
   Arg Arg Ile Ala Leu
   1               5
<210> 38
   <211> 5
   <212> PRT
   <213> Artificial
<220>
   <223> Chemical synthesized
<400> 38
   Arg Arg Lys Ala Leu
   1               5
<210> 39
   <211> 5
   <212> PRT
   <213> Artificial
<220>
   <223> Chemical synthesized
<400> 39
   Arg Arg Leu Ala Leu
   1               5
<210> 40
   <211> 5
   <212> PRT
   <213> Artificial
<220>
   <223> Chemical synthesized
<400> 40
   Arg Arg Met Ala Leu
   1 5
<210> 41
   <211> 5
   <212> PRT
   <213> Artificial
<220>
   <223> Chemical synthesized
<400> 41
   Arg Arg Asn Ala Leu
   1 5
<210> 42
   <211> 5
   <212> PRT
   <213> Artificial
<220>
   <223> Chemical synthesized
<400> 42
   Arg Arg Arg Ala Leu
   1 5
<210> 43
   <211> 5
   <212> PRT
   <213> Artificial
<220>
   <223> Chemical synthesized
<400> 43
   Arg Arg Pro Ala Leu
   1 5
<210> 44
   <211> 5
   <212> PRT
   <213> Artificial
<220>
   <223> Chemical synthesized
<400> 44
   Arg Arg Gln Ala Leu
   1 5
<210> 45
   <211> 4
   <212> PRT
   <213> Artificial
<220>
   <223> Chemical synthesized
<400> 45
   Tyr Trp Arg Tyr
   1
<210> 46
   <211> 5
   <212> PRT
   <213> Artificial
<220>
   <223> Chemical synthesized
<220>
   <221> misc_feature
   <222> (1).. (1)
   <223> Xaa=2-Propynoic acid linker anchoring the azide a-Gal trisacchride
<400> 46
   Xaa Tyr Trp Arg Tyr
   1 5
<210> 47
   <211> 5
   <212> PRT
   <213> Artificial
<220>
   <223> Chemical synthesized
<220>
   <221> misc_feature
   <222> (2)..(2)
   <223> Xaa=2-Propynoic acid linker anchoring the azide a-Gal trisacchride
<400> 47
   Tyr Xaa Trp Arg Tyr
   1 5
<210> 48
   <211> 5
   <212> PRT
   <213> Artificial
<220>
   <223> Chemical synthesized
<220>
   <221> misc_feature
   <222> (3)..(3)
   <223> Xaa=2-Propynoic acid linker anchoring the azide a-Gal trisacchride
<400> 48
   Tyr Trp Xaa Arg Tyr
   1 5
<210> 49
   <211> 5
   <212> PRT
   <213> Artificial
<220>
   <223> Chemical synthesized
<220>
   <221> misc_feature
   <222> (4)..(4)
   <223> Xaa=2-Propynoic acid linker anchoring the azide a-Gal trisacchride
<400> 49
   Tyr Trp Arg Xaa Tyr
   1 5
<210> 50
   <211> 5
   <212> PRT
   <213> Artificial
<220>
   <223> Chemical synthesized
<220>
   <221> misc_feature
   <222> (5)..(5)
   <223> Xaa=2-Propynoic acid linker anchoring the azide a-Gal trisacchride
<400> 50
   Tyr Trp Arg Tyr Xaa
   1 5
<210> 51
   <211> 4
   <212> PRT
   <213> Artificial
<220>
   <223> Chemical synthesized
<220>
   <221> misc_feature
   <222> (1).. (1)
   <223> Xaa=2-Propynoic acid linker anchoring the azide a-Gal trisacchride
<400> 51
   Xaa Trp Arg Tyr
   1
<210> 52
   <211> 4
   <212> PRT
   <213> Artificial
<220>
   <223> Chemical synthesized
<220>
   <221> misc_feature
   <222> (2)..(2)
   <223> Xaa=2-Propynoic acid linker anchoring the azide a-Gal trisacchride
<400> 52
   Tyr Xaa Arg Tyr
   1
<210> 53
   <211> 4
   <212> PRT
   <213> Artificial
<220>
   <223> Chemical synthesized
<220>
   <221> misc_feature
   <222> (3)..(3)
   <223> Xaa=2-Propynoic acid linker anchoring the azide a-Gal trisacchride
<400> 53
   Tyr Trp Xaa Tyr
   1
<210> 54
   <211> 4
   <212> PRT
   <213> Artificial
<220>
   <223> Chemical synthesized
<220>
   <221> misc_feature
   <222> (4)..(4)
   <223> Xaa=2-Propynoic acid linker anchoring the azide a-Gal trisacchride
<400> 54
   Tyr Trp Arg Xaa
   1
<210> 55
   <211> 4
   <212> PRT
   <213> Artificial
<220>
   <223> Chemical synthesized
<400> 55
   Thr Trp Arg Tyr
   1
<210> 56
   <211> 5
   <212> PRT
   <213> Artificial
<220>
   <223> Chemical synthesized
<220>
   <221> misc_feature
   <222> (1).. (1)
   <223> Xaa=2-Propynoic acid linker anchoring the azide a-Gal trisacchride
<400> 56
   Xaa Thr Trp Arg Tyr
   1 5
<210> 57
   <211> 5
   <212> PRT
   <213> Artificial
<220>
   <223> Chemical synthesized
<220>
   <221> misc_feature
   <222> (2).. (2)
   <223> Xaa=2-Propynoic acid linker anchoring the azide a-Gal trisacchride
<400> 57
   Thr Xaa Trp Arg Tyr
   1 5
<210> 58
   <211> 5
   <212> PRT
   <213> Artificial
<220>
   <223> Chemical synthesized
<220>
   <221> misc_feature
   <222> (3)..(3)
   <223> Xaa=2-Propynoic acid linker anchoring the azide a-Gal trisacchride
<400> 58
   Thr Trp Xaa Arg Tyr
   1 5
<210> 59
   <211> 5
   <212> PRT
   <213> Artificial
<220>
   <223> Chemical synthesized
<220>
   <221> misc_feature
   <222> (4)..(4)
   <223> Xaa=2-Propynoic acid linker anchoring the azide a-Gal trisacchride
<400> 59
   Thr Trp Arg Xaa Tyr
   1 5
<210> 60
   <211> 5
   <212> PRT
   <213> Artificial
<220>
   <223> Chemical synthesized
<220>
   <221> misc_feature
   <222> (5)..(5)
   <223> Xaa=2-Propynoic acid linker anchoring the azide a-Gal trisacchride
<400> 60
   Thr Trp Arg Tyr Xaa
   1 5
<210> 61
   <211> 4
   <212> PRT
   <213> Artificial
<220>
   <223> Chemical synthesized
<220>
   <221> misc_feature
   <222> (2)..(2)
   <223> Xaa=2-Propynoic acid linker anchoring the azide a-Gal trisacchride
<400> 61
   Thr Xaa Arg Tyr
   1
<210> 62
   <211> 4
   <212> PRT
   <213> Artificial
<220>
   <223> Chemical synthesized
<220>
   <221> misc_feature
   <222> (3)..(3)
   <223> Xaa=2-Propynoic acid linker anchoring the azide a-Gal trisacchride
<400> 62
   Thr Trp Xaa Tyr
   1
<210> 63
   <211> 4
   <212> PRT
   <213> Artificial
<220>
   <223> Chemical synthesized
<220>
   <221> misc_feature
   <222> (4)..(4)
   <223> Xaa=2-Propynoic acid linker anchoring the azide a-Gal trisacchride
<400> 63
   Thr Trp Arg Xaa
   1
<210> 64
   <211> 4
   <212> PRT
   <213> Artificial
<220>
   <223> Chemical synthesized
<400> 64
   Arg Trp Arg Tyr
   1
<210> 65
   <211> 5
   <212> PRT
   <213> Artificial
<220>
   <223> Chemical synthesized
<220>
   <221> misc_feature
   <222> (1)..(1)
   <223> Xaa=2-Propynoic acid linker anchoring the azide a-Gal trisacchride
<400> 65
   Xaa Arg Trp Arg Tyr
   1 5
<210> 66
   <211> 5
   <212> PRT
   <213> Artificial
<220>
   <223> Chemical synthesized
<220>
   <221> misc_feature
   <222> (2)..(2)
   <223> Xaa=2-Propynoic acid linker anchoring the azide a-Gal trisacchride
<400> 66
   Arg Xaa Trp Arg Tyr
   1 5
<210> 67
   <211> 5
   <212> PRT
   <213> Artificial
<220>
   <223> Chemical synthesized
<220>
   <221> misc_feature
   <222> (3)..(3)
   <223> Xaa=2-Propynoic acid linker anchoring the azide a-Gal trisacchride
<400> 67
   Arg Trp Xaa Arg Tyr
   1 5
<210> 68
   <211> 5
   <212> PRT
   <213> Artificial
<220>
   <223> Chemical synthesized
<220>
   <221> misc_feature
   <222> (4)..(4)
   <223> xaa=2-Propynoic acid linker anchoring the azide a-Gal trisacchride
<400> 68
   Arg Trp Arg Xaa Tyr
   1 5
<210> 69
   <211> 5
   <212> PRT
   <213> Artificial
<220>
   <223> Chemical synthesized
<220>
   <221> misc_feature
   <222> (5)..(5)
   <223> Xaa=2-Propynoic acid linker anchoring the azide a-Gal trisacchride
<400> 69
   Arg Trp Arg Tyr Xaa
   1 5
<210> 70
   <211> 4
   <212> PRT
   <213> Artificial
<220>
   <223> Chemical synthesized
<220>
   <221> misc_feature
   <222> (2)..(2)
   <223> Xaa=2-Propynoic acid linker anchoring the azide a-Gal trisacchride
<400> 70
   Arg Xaa Arg Tyr
   1
<210> 71
   <211> 4
   <212> PRT
   <213> Artificial
<220>
   <223> Chemical synthesized
<220>
   <221> misc_feature
   <222> (3)..(3)
   <223> Xaa=2-Propynoic acid linker anchoring the azide a-Gal trisacchride
<400> 71
   Arg Trp Xaa Tyr
   1
<210> 72
   <211> 4
   <212> PRT
   <213> Artificial
<220>
   <223> Chemical synthesized
<220>
   <221> misc_feature
   <222> (4)..(4)
   <223> Xaa=2-Propynoic acid linker anchoring the azide a-Gal trisacchride
<400> 72
   Arg Trp Arg Xaa
   1
<210> 73
   <211> 5
   <212> PRT
   <213> Artificial
<220>
   <223> Chemical synthesized
<220>
   <221> misc_feature
   <222> (1).. (1)
   <223> Xaa=3-Butynoic acid linker anchoring the azide a-Gal trisacchride
<400> 73
   Xaa Tyr Trp Arg Tyr
   1               5
<210> 74
   <211> 5
   <212> PRT
   <213> Artificial
<220>
   <223> Chemical synthesized
<220>
   <221> misc_feature
   <222> (2)..(2)
   <223> Xaa=3-Butynoic acid linker anchoring the azide a-Gal trisacchride
<400> 74
   Tyr Xaa Trp Arg Tyr
   1               5
<210> 75
   <211> 5
   <212> PRT
   <213> Artificial
<220>
   <223> Chemical synthesized
<220>
   <221> misc_feature
   <222> (3)..(3)
   <223> Xaa=3-Butynoic acid linker anchoring the azide a-Gal trisacchride
<400> 75
   Tyr Trp Xaa Arg Tyr
   1               5
<210> 76
   <211> 5
   <212> PRT
   <213> Artificial
<220>
   <223> Chemical synthesized
<220>
   <221> misc_feature
   <222> (4)..(4)
   <223> Xaa=3-Butynoic acid linker anchoring the azide a-Gal trisacchride
<400> 76
   Tyr Trp Arg Xaa Tyr
   1               5
<210> 77
   <211> 5
   <212> PRT
   <213> Artificial
<220>
   <223> Chemical synthesized
<220>
   <221> misc_feature
   <222> (5)..(5)
   <223> Xaa=3-Butynoic acid linker anchoring the azide a-Gal trisacchride
<400> 77
   Tyr Trp Arg Tyr Xaa
   1 5
<210> 78
   <211> 4
   <212> PRT
   <213> Artificial
<220>
   <223> Chemical synthesized
<220>
   <221> misc_feature
   <222> (1).. (1)
   <223> Xaa=3-Butynoic acid linker anchoring the azide a-Gal trisacchride
<400> 78
   Xaa Trp Arg Tyr
   1
<210> 79
   <211> 4
   <212> PRT
   <213> Artificial
<220>
   <223> Chemical synthesized
<220>
   <221> misc_feature
   <222> (2)..(2)
   <223> Xaa=3-Butynoic acid linker anchoring the azide a-Gal trisacchride
<400> 79
   Tyr Xaa Arg Tyr
   1
<210> 80
   <211> 4
   <212> PRT
   <213> Artificial
<220>
   <223> Chemical synthesized
<220>
   <221> misc_feature
   <222> (3)..(3)
   <223> Xaa=3-Butynoic acid linker anchoring the azide a-Gal trisacchride
<400> 80
   Tyr Trp Xaa Tyr
   1
<210> 81
   <211> 4
   <212> PRT
   <213> Artificial
<220>
   <223> Chemical synthesized
<220>
   <221> misc_feature
   <222> (4)..(4)
   <223> Xaa=3-Butynoic acid linker anchoring the azide a-Gal trisacchride
<400> 81
   Tyr Trp Arg Xaa
   1
<210> 82
   <211> 5
   <212> PRT
   <213> Artificial
<220>
   <223> Chemical synthesized
<220>
   <221> misc_feature
   <222> (1)..(1)
   <223> Xaa=3-Butynoic acid linker anchoring the azide a-Gal trisacchride
<400> 82
   Xaa Thr Trp Arg Tyr
   1 5
<210> 83
   <211> 5
   <212> PRT
   <213> Artificial
<220>
   <223> Chemical synthesized
<220>
   <221> misc_feature
   <222> (2)..(2)
   <223> Xaa=3-Butynoic acid linker anchoring the azide a-Gal trisacchride
<400> 83
   Thr Xaa Trp Arg Tyr
   1 5
<210> 84
   <211> 5
   <212> PRT
   <213> Artificial
<220>
   <223> Chemical synthesized
<220>
   <221> misc_feature
   <222> (3)..(3)
   <223> Xaa=3-Butynoic acid linker anchoring the azide a-Gal trisacchride
<400> 84
   Thr Trp Xaa Arg Tyr
   1 5
<210> 85
   <211> 5
   <212> PRT
   <213> Artificial
<220>
   <223> Chemical synthesized
<220>
   <221> misc_feature
   <222> (4)..(4)
   <223> Xaa=3-Butynoic acid linker anchoring the azide a-Gal trisacchride
<400> 85
   Thr Trp Arg Xaa Tyr
   1 5
<210> 86
   <211> 5
   <212> PRT
   <213> Artificial
<220>
   <223> Chemical synthesized
<220>
   <221> misc_feature
   <222> (5)..(5)
   <223> Xaa=3-Butynoic acid linker anchoring the azide a-Gal trisacchride
<400> 86
   Thr Trp Arg Tyr Xaa
   1 5
<210> 87
   <211> 4
   <212> PRT
   <213> Artificial
<220>
   <223> Chemical synthesized
<220>
   <221> misc_feature
   <222> (1)..(1)
   <223> Xaa=3-Butynoic acid linker anchoring the azide a-Gal trisacchride
<400> 87
   Xaa Trp Arg Tyr
   1
<210> 88
   <211> 4
   <212> PRT
   <213> Artificial
<220>
   <223> Chemical synthesized
<220>
   <221> misc_feature
   <222> (2)..(2)
   <223> Xaa=3-Butynoic acid linker anchoring the azide a-Gal trisacchride
<400> 88
   Thr Xaa Arg Tyr
   1
<210> 89
   <211> 4
   <212> PRT
   <213> Artificial
<220>
   <223> Chemical synthesized
<220>
   <221> misc_feature
   <222> (3)..(3)
   <223> Xaa=3-Butynoic acid linker anchoring the azide a-Gal trisacchride
<400> 89
   Thr Trp Xaa Tyr
   1
<210> 90
   <211> 4
   <212> PRT
   <213> Artificial
<220>
   <223> Chemical synthesized
<220>
   <221> misc_feature
   <222> (4)..(4)
   <223> Xaa=3-Butynoic acid linker anchoring the azide a-Gal trisacchride
<400> 90
   Thr Trp Arg Xaa
   1
<210> 91
   <211> 5
   <212> PRT
   <213> Artificial
<220>
   <223> Chemical synthesized
<220>
   <221> misc_feature
   <222> (1)..(1)
   <223> Xaa=3-Butynoic acid linker anchoring the azide a-Gal trisacchride
<400> 91
   Xaa Arg Trp Arg Tyr
   1 5
<210> 92
   <211> 5
   <212> PRT
   <213> Artificial
<220>
   <223> Chemical synthesized
<220>
   <221> misc_feature
   <222> (2)..(2)
   <223> Xaa=3-Butynoic acid linker anchoring the azide a-Gal trisacchride
<400> 92
   Arg Xaa Trp Arg Tyr
   1 5
<210> 93
   <211> 5
   <212> PRT
   <213> Artificial
<220>
   <223> Chemical synthesized
<220>
   <221> misc_feature
   <222> (3)..(3)
   <223> Xaa=3-Butynoic acid linker anchoring the azide a-Gal trisacchride
<400> 93
   Arg Trp Xaa Arg Tyr
   1 5
<210> 94
   <211> 5
   <212> PRT
   <213> Artificial
<220>
   <223> Chemical synthesized
<220>
   <221> misc_feature
   <222> (4)..(4)
   <223> Xaa=3-Butynoic acid linker anchoring the azide a-Gal trisacchride
<400> 94
   Arg Trp Arg Xaa Tyr
   1 5
<210> 95
   <211> 5
   <212> PRT
   <213> Artificial
<220>
   <223> Chemical synthesized
<220>
   <221> misc_feature
   <222> (5)..(5)
   <223> Xaa=3-Butynoic acid linker anchoring the azide a-Gal trisacchride
<400> 95
   Arg Trp Arg Tyr Xaa
   1 5
<210> 96
   <211> 4
   <212> PRT
   <213> Artificial
<220>
   <223> Chemical synthesized
<220>
   <221> misc_feature
   <222> (1)..(1)
   <223> Xaa=3-Butynoic acid linker anchoring the azide a-Gal trisacchride
<400> 96
   Xaa Trp Arg Tyr
   1
<210> 97
   <211> 4
   <212> PRT
   <213> Artificial
<220>
   <223> Chemical synthesized
<220>
   <221> misc_feature
   <222> (2)..(2)
   <223> Xaa=3-Butynoic acid linker anchoring the azide a-Gal trisacchride
<400> 97
   Arg Xaa Arg Tyr
   1
<210> 98
   <211> 4
   <212> PRT
   <213> Artificial
<220>
   <223> Chemical synthesized
<220>
   <221> misc_feature
   <222> (3)..(3)
   <223> Xaa=3-Butynoic acid linker anchoring the azide a-Gal trisacchride
<400> 98
   Arg Trp Xaa Tyr
   1
<210> 99
   <211> 4
   <212> PRT
   <213> Artificial
<220>
   <223> Chemical synthesized
<220>
   <221> misc_feature
   <222> (4).. (4)
   <223> Xaa=3-Butynoic acid linker anchoring the azide a-Gal trisacchride
<400> 99
   Arg Trp Arg Xaa
   1
<210> 100
   <211> 5
   <212> PRT
   <213> Artificial
<220>
   <223> Chemical synthesized
<220>
   <221> misc_feature
   <222> (1)..(1)
   <223> Xaa=4-Pentynoic acid linker anchoring the azide a-Gal trisacchride
<400> 100
   Xaa Tyr Trp Arg Tyr
   1               5
<210> 101
   <211> 5
   <212> PRT
   <213> Artificial
<220>
   <223> Chemical synthesized
<220>
   <221> misc_feature
   <222> (2)..(2)
   <223> Xaa=4-Pentynoic acid linker anchoring the azide a-Gal trisacchride
<400> 101
   Tyr Xaa Trp Arg Tyr
   1               5
<210> 102
   <211> 5
   <212> PRT
   <213> Artificial
<220>
   <223> Chemical synthesized
<220>
   <221> misc_feature
   <222> (3)..(3)
   <223> Xaa=4-Pentynoic acid linker anchoring the azide a-Gal trisacchride
<400> 102
   Tyr Trp Xaa Arg Tyr
   1               5
<210> 103
   <211> 5
   <212> PRT
   <213> Artificial
<220>
   <223> Chemical synthesized
<400> 103
   Tyr Trp Arg Xaa Tyr
   1 5
<210> 104
   <211> 5
   <212> PRT
   <213> Artificial
<220>
   <223> Chemical synthesized
<400> 104
   Tyr Trp Arg Tyr Xaa
   1 5
<210> 105
   <211> 4
   <212> PRT
   <213> Artificial
<220>
   <223> Chemical synthesized
<220>
   <221> misc_feature
   <222> (1).. (1)
   <223> Xaa=4-Pentynoic acid linker anchoring the azide a-Gal trisacchride
<400> 105
   Xaa Trp Arg Tyr
   1
<210> 106
   <211> 4
   <212> PRT
   <213> Artificial
<220>
   <223> Chemical synthesized
<220>
   <221> misc_feature
   <222> (2)..(2)
   <223> Xaa=4-Pentynoic acid linker anchoring the azide a-Gal trisacchride
<400> 106
   Tyr Xaa Arg Tyr
   1
<210> 107
   <211> 4
   <212> PRT
   <213> Artificial
<220>
   <223> Chemical synthesized
<220>
   <221> misc_feature
   <222> (3)..(3)
   <223> Xaa=4-Pentynoic acid linker anchoring the azide a-Gal trisacchride
<400> 107
   Tyr Trp Xaa Tyr
   1
<210> 108
   <211> 4
   <212> PRT
   <213> Artificial
<220>
   <223> Chemical synthesized
<220>
   <221> misc_feature
   <222> (4)..(4)
   <223> Xaa=4-Pentynoic acid linker anchoring the azide a-Gal trisacchride
<400> 108
   Tyr Trp Arg Xaa
   1
<210> 109
   <211> 5
   <212> PRT
   <213> Artificial
<220>
   <223> Chemical synthesized
<220>
   <221> misc_feature
   <222> (1)..(1)
   <223> Xaa=4-Pentynoic acid linker anchoring the azide a-Gal trisacchride
<400> 109
   Xaa Thr Trp Arg Tyr
   1               5
<210> 110
   <211> 5
   <212> PRT
   <213> Artificial
<220>
   <223> Chemical synthesized
<220>
   <221> misc_feature
   <222> (2)..(2)
   <223> Xaa=4-Pentynoic acid linker anchoring the azide a-Gal trisacchride
<400> 110
   Thr Xaa Trp Arg Tyr
   1 5
<210> 111
   <211> 5
   <212> PRT
   <213> Artificial
<220>
   <223> Chemical synthesized
<220>
   <221> misc_feature
   <222> (3)..(3)
   <223> Xaa=4-Pentynoic acid linker anchoring the azide a-Gal trisacchride
<400> 111
   Thr Trp Xaa Arg Tyr
   1 5
<210> 112
   <211> 5
   <212> PRT
   <213> Artificial
<220>
   <223> Chemical synthesized
<220>
   <221> misc_feature
   <222> (4).. (4)
   <223> Xaa=4-Pentynoic acid linker anchoring the azide a-Gal trisacchride
<400> 112
   Thr Trp Arg Xaa Tyr
   1 5
<210> 113
   <211> 5
   <212> PRT
   <213> Artificial
<220>
   <223> Chemical synthesized
<220>
   <221> misc_feature
   <222> (5)..(5)
   <223> Xaa=4-Pentynoic acid linker anchoring the azide a-Gal trisacchride
<400> 113
   Thr Trp Arg Tyr Xaa
   1 5
<210> 114
   <211> 4
   <212> PRT
   <213> Artificial
<220>
   <223> Chemical synthesized
<220>
   <221> misc_feature
   <222> (1)..(1)
   <223> Xaa=4-Pentynoic acid linker anchoring the azide a-Gal trisacchride
<400> 114
   Xaa Trp Arg Tyr
   1
<210> 115
   <211> 4
   <212> PRT
   <213> Artificial
<220>
   <223> Chemical synthesized
<220>
   <221> misc_feature
   <222> (2)..(2)
   <223> Xaa=4-Pentynoic acid linker anchoring the azide a-Gal trisacchride
<400> 115
   Thr Xaa Arg Tyr
   1
<210> 116
   <211> 4
   <212> PRT
   <213> Artificial
<220>
   <223> Chemical synthesized
<220>
   <221> misc_feature
   <222> (3)..(3)
   <223> Xaa=4-Pentynoic acid linker anchoring the azide a-Gal trisacchride
<400> 116
   Thr Trp Xaa Tyr
   1
<210> 117
   <211> 4
   <212> PRT
   <213> Artificial
<220>
   <223> Chemical synthesized
<220>
   <221> misc_feature
   <222> (4)..(4)
   <223> Xaa=4-Pentynoic acid linker anchoring the azide a-Gal trisacchride
<400> 117
   Thr Trp Arg Xaa
   1
<210> 118
   <211> 5
   <212> PRT
   <213> Artificial
<220>
   <223> Chemical synthesized
<220>
   <221> misc_feature
   <222> (1)..(1)
   <223> Xaa=4-Pentynoic acid linker anchoring the azide a-Gal trisacchride
<400> 118
   Xaa Arg Trp Arg Tyr
   1 5
<210> 119
   <211> 5
   <212> PRT
   <213> Artificial
<220>
   <223> Chemical synthesized
<220>
   <221> misc_feature
   <222> (2)..(2)
   <223> Xaa=4-Pentynoic acid linker anchoring the azide a-Gal trisacchride
<400> 119
   Arg Xaa Trp Arg Tyr
   1 5
<210> 120
   <211> 5
   <212> PRT
   <213> Artificial
<220>
   <223> Chemical synthesized
<220>
   <221> misc_feature
   <222> (3)..(3)
   <223> Xaa=4-Pentynoic acid linker anchoring the azide a-Gal trisacchride
<400> 120
   Arg Trp Xaa Arg Tyr
   1               5
<210> 121
   <211> 5
   <212> PRT
   <213> Artificial
<220>
   <223> Chemical synthesized
<220>
   <221> misc_feature
   <222> (4).. (4)
   <223> Xaa=4-Pentynoic acid linker anchoring the azide a-Gal trisacchride
<400> 121
   Arg Trp Arg Xaa Tyr
   1               5
<210> 122
   <211> 5
   <212> PRT
   <213> Artificial
<220>
   <223> Chemical synthesized
<220>
   <221> misc_feature
   <222> (5)..(5)
   <223> Xaa=4-Pentynoic acid linker anchoring the azide a-Gal trisacchride
<400> 122
   Arg Trp Arg Tyr Xaa
   1               5
<210> 123
   <211> 4
   <212> PRT
   <213> Artificial
<220>
   <223> Chemical synthesized
<220>
   <221> misc_feature
   <222> (1)..(1)
   <223> Xaa=4-Pentynoic acid linker anchoring the azide a-Gal trisacchride
<400> 123
   Xaa Trp Arg Tyr
   1
<210> 124
   <211> 4
   <212> PRT
   <213> Artificial
<220>
   <223> Chemical synthesized
<220>
   <221> misc_feature
   <222> (2).. (2)
   <223> Xaa=4-Pentynoic acid linker anchoring the azide a-Gal trisacchride
<400> 124
   Arg Xaa Arg Tyr
   1
<210> 125
   <211> 4
   <212> PRT
   <213> Artificial
<220>
   <223> Chemical synthesized
<220>
   <221> misc_feature
   <222> (3)..(3)
   <223> Xaa=4-Pentynoic acid linker anchoring the azide a-Gal trisacchride
<400> 125
   Arg Trp Xaa Tyr
   1
<210> 126
   <211> 4
   <212> PRT
   <213> Artificial
<220>
   <223> Chemical synthesized
<220>
   <221> misc_feature
   <222> (9)..(4)
   <223> Xaa=4-Pentynoic acid linker anchoring the azide a-Gal trisacchride
<400> 126
   Arg Trp Arg Xaa
   1

## Description

### BACKGROUND OF THE INVENTION

### Field of the invention

The present invention relates to quantitative and quantity aspects of array synthesis and array uses as a device for high capacity producing synthetic molecules for off-array surface applications and as an assay device for on-array surface applications.

The present invention specifically relates to methods for controlling surface molecular densities of an array at different array sites to create a density-variation array. These arrays will be applied to on-surface or off-surface applications, such as high throughput quantitative measurements for protein bindings, nucleic acid bindings, and sensitive biomarker detections. Arrays containing density-variation sites are expected to be used for obtaining binding and dissociation curves of molecular complexes, and to be especially valuable for clinical and medical applications where accuracy of the detection and measurements are more demanding and significant

The present invention also relates to the field of modified array surface for parallel array synthesis to increase the amount of the molecules synthesized by a factor of at least 10. These high capacity arrays will be of use for on- and off-surface applications in the areas of genome-wide and proteome-wide assays. The molecular modifications also provide bulk material supplies for preparation of nanoarrays, single molecule arrays, structural studies or sequence analysis of nucleic acids, proteins, polysaccharides, other types of biopolymers and chimeric biopolymers, affinity purification, drug screening, biomarker detection, genetic analysis, profiling of nucleic acids, proteins or other types of biomolecules, clinical diagnosis and patient sample analysis, and forming sequence libraries or bead-based sequence libraries of nucleic acids, proteins, peptides, or other types of molecular libraries.

The present invention also relates to methods for synthesis of array molecules containing modifications. The array of molecules Synthesized will be used for on- and off-surface applications in the areas of genome-wide and proteome-wide assays. The molecular modifications provide unique ligand arrays for analytes of not only proteins, nuclei acids, carbohydrates, but also cell or cellular organisms. The molecular modifications also provide bulk material supplies for preparation of nanoarrays, single molecule arrays, structural studies or sequence analysis of nucleic acids, proteins, polysaccharides, other types of biopolymers and chimeric biopolymers, affinity purification, drug screening, biomarker detection, genetic analysis, profiling of nucleic acids, proteins or other types of biomolecules, clinical diagnosis and patient sample analysis, and forming sequence libraries or bead-based sequence libraries of nucleic acids, proteins, peptides, or other types of molecular libraries.

### Description of Related Art

The rapid development of the genomics (genomics, broadly represents herein the subject areas related to cellular molecules and their interactions, including nucleic acids, proteins, peptides, carbohydrates, lipids, metabolic molecules, and other functional molecules) and related sciences have constantly created need of miniaturization technologies. It is well known that each round of miniaturization of an existing technology requires major advancement in methods and devices. Today, the most talked human genome of three billion bases and more than 100k proteins and their isomers is a clear example of the needs. For this large number of genetic codes and the products of the genetic codes, there have been mounting information to show that their analysis (finding out what are the codes) and profiling (measuring how many and how much of them are present) are essential for understanding of the basics of the cellular molecular relationships, identify an individual's genetic proposition, health condition, disease condition, drug response, immuno-responsiveness.

Analysis and profiling of biomolecules require specificity and sensitivity. While these are now common practices in many forms of assays, these are a single analysis or reaction at a time. In an ideal situation, at least one component of the assay has no competition (or cross reaction possibility). The results thus obtained are likely to be specific. For instance, in analyzing RNA using Northern Blotting, a specific probe will be selected and used to hybridize with the target molecule in the absence of the competition of other non-specific probes. In another example, a protein kinase assay kit would contain one protein kinase and one peptide as the substrate of the enzyme or a protein kinase kit would contain one protein kinase, one peptide and one inhibitor peptide for measurements of enzyme activities or inhibitory effects, respectively. The results of conventional assays tend to be more reliable due to their simplified experimental conditions. These experiments do not demand many replicates or complete binding curves for the specificity of the measurements.

High throughput assays means simultaneously processing many different samples and/or a comprehensive assay which will also simultaneously analyze many samples of a biological system. The current microwell plates run 96, 384, 1536 assays in parallel in volumes of microliters to submicroliters. The use of these microwell plates requires liquid handling robotics, plate loaders, and large storage spaces. The high throughput assays using microwell plates will not be practical if the assays are a genome-wide scale, since there will be high cost and impractical material consumption problems. For instance, if a population of one million people were tested for 10,000 important genetic regions, the total experiment number would be 10 billion. If each experiment consumes 10 microliter reagent/solution, a total of 100,000 liter of assay solution will be needled. Clearly, even though now we already have rich information about human genomes, genome association with diseases, single nucleotide polymorphism (SNP), DNA methylation, histone modification, DNA insertion/delection/transposition, and other types of genome information (see National Center of Bioinformatics, NCBI, http://www.ncbi.nlm.nih.gov/). only few assays are affordable. It is therefore a necessary step that the assay volumes are reduced and the assay devices are miniaturized.

High throughput assays are also widely used for whole cell and living cell analysis, such as analysis of apoptosis, protein expression or enzymatic activities in cells, cell-based phenotype-based drug screening, cyto-toxicity, and cell cycles. Current platforms, such as Beckman Coulter's Cell Lab™, support sample cups, micro centrifuge tubes and 24~, 96- or 384~well plates, or flow cytometry, analyzing more than 100,000 events per second. It is now possible to simultaneously analyzing multiple variables and parameters using fluorescent labels in high content screening assays. These technological advancements and the availability of modern optical microscopic instruments, such as confocal and the total internal reflection fluorescence (TIRF) Microscopy, are powerful tools for deepening of our understanding of in vivo systems. There are clear needs of improvements over the current systems. For instance, often even one event in a large population, such as a thousand or a million, can be of diagnostic or research significance. It is desirable to enrich the cells/events that are of major interest and analyze these cells/events into great details using appropriate instruments or methods, such as those discussed above.

Microarrays are reaction and assay devices which were developed in the last two decades and increasingly used as high throughput tools. Microarrays have dimensions in microns for each array site and a capacity of thousands to one million assay sites on a square centimeter area. Some assays may use picoliters or less reaction solution per assay site. Microarrays do not use liquid handling instruments as used for microwell plates and for the first time to allow genome-wide assays run in high throughput mode with significant reduction of reaction solutions used.

While one approach of high throughput as described above is to run parallel single reaction assays, many multiplex reaction assays involving a mixture of assay molecules and analytes have been developed.

Multiplex assays have been done using combinatorial bead libraries with each bead containing one type of molecules, such as peptides, small organic molecule, oligonucleotides, or other types of assay molecules

A plethora of miniaturizing devices have been described and a common feature of the technology advancement is to enable assays using nanoliter or smaller volumes. However, the degree of difficulties of having diverse contents in a miniaturizing device increases rapidly as the size of feature reduces. This has been the barrier for many research and development devices to become commercial products of low cost, easy to use, and diverse applications.

One method of creating contents of assay devices is to pre-synthesize or prepare a large number of different molecules and place them at different reaction sites in the assay device such as cDNA or spotting DNA oligonucleotide arrays. As the number of assays increases and the amount of materials decreases, the front costs of the material preparation become unbearable and some times it is impossible to synthesize or prepare the large number of different molecules required for the assay. Therefore, the method is limited to arrays of a few hundreds of features, such as antibody arrays², carbohydrate arrays², or small molecule arrays⁴, but for DNA oligonucleotides, the number of feature are in thousands to less than thirty thousands. For assays of nanoliter or smaller volumes, robotic instruments face many technical challenges and they do not provide sufficient precision for precisely deliver (or spot) the assay materials to the reaction sites. The instrument which could provide such capabilities will be prohibitively expensive for routine laboratorial use.

Alternatively, methods have been developed for in situ synthesis of high density arrays of molecules^{5 6 7 8 9 10} i.e., molecules are directly synthesized on a solid surface by stepwise additions of monomers or building blocks. These methods overcome the difficulties for pre-synthesis and have been widely used for DNA oligonucleotide microarray-based assays (e.g. Affymetrix's GeneChip™, Nimblegen DNA microarray, Agilent's ink-jet DNA microarray, Atactic Technologies/LC Sciences' µParaflo™ DNA/RNA Microarrays) and peptide microarray-based assays (JPT SPOT synthesis on nitrocellulose fabric materials, Atactic Technologies' µParaflo™ peptide microarrays).

Most high density arrays contain standard DNA oligonucleotides due to the limitation of the synthesis methods which require synthesis monomers specially made for photolabile deprotection-based synthesis^{7 8}. For any modifications on the standard chemical moieties, a new photolabile group protected monomer must be prepared, and the corresponding synthesis for making arrays will need to be optimized. The extensive effort involved for a new type of sequences and their unknown outcomes associated with modifications of the standard synthesis make the photolabile protecting group-based method limited to arrays containing standard DNA oligonucleotides. Array synthesis using ink-jet instrument relies on conventional chemistry and the chemistry per se can allow incorporation of modified residues. However, such synthesis will require a compatible synthesis instrument to accommodate additional reagents and these requirements also make the ink-jet method limited to arrays containing standard DNA oligonucleotides.

A µParaflo array synthesis method^{5 6} overcomes the above limitations. The method is based on conventional chemistries, such dimethoxytrityl (DMT) nucleophosphoramidite (amidite) chemistry for DNA and RNA oligonucleotides, tert-butyloxycarbonyl (Boc) chemistry or 9-Fluorenylmethoxycarbonyl (Fmoc) chemistry for peptide chemistry, and light-activated by photogenerated reagents, such as an acid or a base, for synthesis of DNA and RNA oligonucleotides and peptides^{11 12 13 14 15}. The method incorporates modifications into array molecules by directly using commercially available monomers, and diverse arrays containing modified residues have been synthesized. The modifications of oligonucleotides or peptides confer them novel binding properties or enzymatic activities. Such arrays have important applications in not only research but also diagnostics, drug screening, disease management, and other areas.

There is a great need in the fields of genomics and proteomics where assays should be performed in a number of millions on a nanoliter or smaller volumes, for parallel synthesis of molecules on smaller scales than the current 96-well plate synthesis (Illumina/Invitrogen). It is also highly desirable for a technology to enable the synthesis of diverse molecules, such as modified oligonucleotides, modified peptides, molecules with tags, on a high density array synthesis device for the various detection, quantitation, and lead-compound development off-array surface applications. In the last decade, array as a format of high throughput bioassays has been well-accepted but most of the in situ parallel synthesis array methods (GeneChip® of Affymetrix, Nimblegen, Febit, Agilent, Combimatrix) are limited to making standard DNA oligonucleotides without modifications. Arrays made for bioassays do not provide the synthesis quality or a means required for obtaining sufficient quantities and removing the molecules synthesized from the array surface for use in the subsequent reactions, such as making nanoarrays, single molecule arrays, genome-wide sequence specific primers for genomic DNA amplifications, or genome-wide target specific probes for DNA or RNA sequences. Recently, a uPicoArray reactor was described for synthesis of thousands of DNA oligonucleotides in parallel, removing the product, and assembling large DNA constructs^{17 18}. The DNA constructs were protein expression vectors for generation of proteins of natural types or by design. The DNA synthesis using miniaturized device reduces the cost by a factor of at least 30 times and increase throughput by a factor of at least ten. There is no need for liquid robotic instruments for handling, the samples. Although it is possible to recover each type of molecules synthesized individually, many applications of multiplex reactions require molecular libraries and thus a large number of different molecules are used as a mixture or subgroups of mixtures.

In the area of diversify synthesis of array molecules, there have been great interests in glycol-conjugates, such as glycopeptides, and glycan analogs due to their important roles in understanding carbohydrate mediated cellular activities, cell migration, cell-cell interactions, cell-protein interactions, protein-protein interactions³. These interactions are implicated in a number of pathogenic, immunogenic pathways that are responsible for infectious diseases, ill-elicitation of immunoreponses, fertilization, embryogenesis, lymphocyte trafficking, tumor genesis, and cancer metastasis. Therefore, glycosylated peptides are of the potential as vaccine, therapeutic, and model compounds. To date, glycopeptide-based assays rely on traditional immunological methods at low throughput, but there are a large number of structural isomers of these compounds. Our understanding of this complex group of compounds is rather limited. The synthesis of arrays of glycopeptides and glycosyl modified peptides can accelerate the studies in this important area and development of glycopeptide-based agents for assay, diagnosis, and therapeutic applications.

Published Applications EP-A-1645 639, US 2002/039728 A1, EP-A-1 081 163 and US 2003/198943 A1 disclose arrays in which there may be inherent and unpredictable variability in the density of the molecules on the arrays.

One distinct advantage of assays using an addressable array format compared to a random array is that there are tractable assay sites as references, positive or negative controls, and other types of controls and replicates of data points for data processing and analysis. These are necessary features for ensuring high quality and reliable assay results. Performing functions such as quality control analysis of synthesis or assays, baseline signal correction, normalization of the signal intensities within the array or of different data sets for generation of reproducible results and deriving quantitative results are depending on these features. On addressable arrays, it is always possible to design and test comparison data sets to exclude the possibilities of false positive and/or negative signals. The synthetic bead molecular libraries without a decode mechanism cannot meet these requirements.

### SUMMARY OF THE INVENTION

The invention provides a solid surface comprising a density-variation molecular array according to claim 1.

### BRIEF DESCRIPTION OF THE FIG.S AND DRAWINGS

FIG. 1 illustrates the concept of density-variation reaction or assay sites in a molecular array.
FIG. 2 illustrates a set of density-variation array sites.
FIG. 3 is a Scheme for synthesis of surface molecules on array sites of varied-densities.
FIG. 4 is a bar-graph of the fluorescent signals of the epitope peptide YPYDVPDYA synthesized on varied-density array sites.
FIG. 5 is an exemplary molecular density-variation array sites shown as a fluorescent image after directly labeling of the molecules synthesized
FIG. 6 is an exemplary molecular density-variation array sites shown as a bar-graph of the measurements from antibody binding to the molecules synthesized.
FIG. 7 illustrates an array of protein kinase substrate peptides and the detection of phosphorylation by phospho-specific binding agent
FIG. 8 illustrates a protein kinase substrate peptide array
FIG. 9 illustrates a protein kinase substrate peptide array containing density-variation sites
FIG. 10 illustrates a plot of the measurements of protein phosphorylation of a protein kinase substrate peptide array containing density-variation array sites
FIG. 11 illustrates a plot of the chain-length dependence of the correction factor for the binding data on a peptide array for compensabon of synthesis deficiency which negatively affect the binding signal intensities.
FIG. 12 displays the images of a portion of a microfluidic array chip (A) Image has empty microfluidic chambers, (B) image has the chambers filled with immobilized beads for synthesis.
FIG. 13 is a microscopy image (white light) of oligonucleotides synthesized on glass plates containing immobilized TentaGel beads The image was taken after the oligonucleotide nucleotide synthesis reaction cycles
FIG. 14 illustrates synthesis on bead-loaded array sites and bead array sites may be density-variation sites
FIG. 15 illustrates the Azide alkyne Huisgen cycloaddition (click chemistry) reaction.
FIG. 16 illustrates the synthesis of bioconjugate polymer arrays using azide alkyne Huisgen cycloaddition (click chemistry) reaction.
FIG. 17 displays the image of anti-Gal antibody binding on a glycosylated peptide array
FIG. 18 illustrates synthesis of bead-modified molecular array

### DETAILED DESCRIPTION OF THE INVENTION

Unless defined otherwise, all technical and scientific terms used herein have the same meaning as commonly understood by one of ordinary skill in the art to which this invention belongs. Still, certain elements are defined below for the sake of clarity and ease of reference

### Definition

Definitions as used herein, the following terms and phrases shall have the meanings set forth bellow. Unless defined otherwise, all technical and scientific terms used herein have the same meaning as commonly understood to one of ordinary skill in the art.

The singular forms "a," "an," and "the" include plural reference unless the context clearly dictates Otherwise.

The following terms are intended to have the following general meaning as defined in the content after the "dash line" as they are used herein:

Activate - In chemical reaction, this means to reduce the energy of a chemical reaction to induce it to occur.

Addressable array - An array on which the identities and specific locations of the molecules are known

An array of molecules - A plurality of mostly different molecules at different locations (spots) of a surface, the low, medium, high or very high densities of an array is determined by both total number of spots on the array and number of spots in a unit area. Currently, an array of several thousands molecules in a square centimeter area is high density; very-high or ultra-high arrays has millions of molecules on a slide (1"x3"). Arrays have different properties which are largely depending on the method of making the array.

Analyte - A substance or a compound that is an object of detection or analysis. An analyte of an array experiments may be a protein, a protein solution, a biological protein samples such as cell lysate, a nucleic acid sequences, a nucleic acid solution, a biological nucleic acid sample such as a genomic DNA or total RNA, treated for array measurements.

Array control sites, reference sites - An array contains a number of sites which are not for sample analysis but for technical quality assessment, such as synthesis, binding, etc.

Array layout - A two dimensional map of an addressable array, with the location and sequence identity written in a file.

Array sites - discrete surface areas on a surface there are multiple sites.

Array, chip - These terms are used interchangeably to refer to a set of sites sharing a common space, the number of sites is usually at least two in a row and at least two in a column; the upper limit of the number of sites per row or per column or per array is set by the dimension for accommodation of a three atom molecule; the sites have a unit density of at least nine per square centimeter and the upper limit of the number of sites per unit area is set by the dimension for accommodation of a three atom molecule; the sites of an array may be but need not to be regularly located on the surface. Each array has a predetermined format, such as planner glass plate with or without array site boundaries, Each site on an array also called features. The molecules on an individual site need not to have the same chemical structures important array properties include array site geometry surface, flow through cell array,chamber array Array subset ill array in a restricted area (array in 96-well)

Assay molecules - Molecules that are used for assays assay molecules are used for analysis of an analytes

Assay sample - A sample containing analyte to be assayed

Bead-array - An array with its content delivered by beads

Beads, particles microsphere, nanoparticles, nanobeads - These terms are used interchangeably to refer to small objects (in dimensions of um to nm) Bead or beads are often used in the present description. Beads can be made from but not limited to, plastics, ceramics glass polystyrene, methylstyrene acrylic polymers, paramagnetic material, thoria sol, carbon graphited titanium dioxide, latex or cross-linked dextrans such as Sepharose, cellulose nylon, cross-linked micelles and Teflon Beads may or may not be spherical, may be elongated, may or may not porous, may or may not be surface coated may or may not be contain functional groups such as NH2 or OH, may or may not be coasted on surface, may or may not be having optical properties, magnetic properties, affinity purification properties

Binding/dissociation curve - Plots of the signal of a binding event as a function of molecule concentration wherein molecule is an analyte or an analytes interacting ligand.

Building block monomer - The terms are used interchangeably, it is more often to use "monomer" for nucleophosphoramidites or protected amino acids.

Carbohydrate, saccharide, oligosaccharide, polysaccharide, glycan - These terms used interchangeably to refer to the carbohydrate moieties, or molecules

Chimera or chimeric molecules - A compound containing at least two groups, motifs, sequences, or moieties from different common families of molecules, examples of a chimera or a chimeric molecule include a chimeric DNA-RNA oligonucleotide, a chimeric peptide from two different proteins, or a chimeric carbohydrate-peptide.

Cleavage, conjugate - To break or form a chemical bond

Conjugate - A chemical bond formed between to molecular moieties.

Dendrimers - molecules which has a branched structure, especially used in this invention dendrimers have multiple branch arms which can be functionalized to multiply the number of growing chain of a synthetic molecule.

Dimension of a three atom molecule - An example of the molecule is water (H-O-H), whose wider dimension is 2.75 angstrom.

DNA array - DNA oligonucleotide array, RNA oligonucleotide array, DNA/RNA oligonucleotide array, RNA array are used interchangeably for arrays of nucleic acids and oligonucleotides

Functionalized surface synthesis initiation moieties - A surface is or can be activated for chemical reactions Often, the functional groups are amino or hydroxyl groups or protect amino or hydroxyl groups

Glycosylated peptide array - Modification of peptides by adding carbohydrates to them

High capacity array - Array as a synthesizer that can deliver more molecules (pmol or more per array site) than a regular array (synthesizing fmol or less per array site).

Hybridize, wash, strip - Nucleic acid hybridization experimental steps hybridization describes the association process of two complementary strands

Immobilization - Reacting of a molecule with surface and forming a chemical bond between them

In situ parallel synthesis - Simultaneous synthesis from de novo of many sequences molecular arrays

Linker surface linker, spacer - "Linker" and 'spacer' are anchoring groups that anchor or tether two molecular moieties; the chain length of a linker or spacer is determined by their linear rotable bonds linker also defines the molecular moiety anchoring the solid support with the first molecule on surface; amino or hydroxyl silanes are often use as linkers on glass surface, examples of spacer include, but are not limited to, ethyleneglycol polymer, alkyl, molecules containing branch side chains dendnmers oligonucleotides, peptides, peptditomimetics

Modified nucleotide, modified peptide, modified carbohydrate - A compound which contains chemical moieties that is different from or additional to those of their natural counterparts

Multiplex reaction, simplex reaction - assays or reactions involving more than two participating molecules such as PCR reaction with ten templates and 20 primers

Nanoparticles (nanorods nanoshells nanocrystals, colloidal particulates)

Neutralizing reagent, Sensitizer, Stabilizer - these are synthesis agents which neutralize acid or base in reaction solution transfer irradiation energy, and trapping radical species

Nucleic acid - a polymeric form of nucleotides, either ribonucleotides and/or deoxyribonucleotides or a modified form of either type of nucleotide. The terms should also be understood to include as equivalents, analog of either RNA or DNA made from nucleotide analogs, and as applicable to the embodiment being described, single-stranded (such as sense or antisense) and double-stranded polynucleotides

Oligo - when used in combination with a chemical moiety name, such as oligonucleotides, oligosaccharides, means a short stretch (i.e., 2, 3, a few, to several hundreds of residues) of the chain of the chemical moiety.

Oligos - An breviation for oligonucleotides -

On-array surface, on-surface versus off-array surface, off-surface application of array molecules - Applications using array molecules on surface; or use chemical or enzymatic means to remove molecules on surface and recover them for other applications such as use as an oligonucleotide mixture for mutagenesis or DNA synthesis.

Peptide - Oligomer made from amide linkages of 20 natural amino acids.

Peptide array - A surface area containing more than 96 peptide sequences immobilized in a square centimeter area

PGA-P, PGB-P, photogenerated acid precursor or phostogenerate base precursor. photogenerated acid (PGA), photogenerated base (PGB), photolabile group protected monomer - These are parallel synthesis agents, activated by light irradiation.

Poly - when used in combination with a chemical moiety name, such as polynucleotides, polysaccharides, means a long stretch (i.e., arbitrarily 50, 51, hundreds, thousands, and longer stretch of residues) of the chain of the chemical moiety.

Predetermined an objective which is planed, arranged before the experiment

Protecting group, protection and deprotection - These terms refer to chemical moieties that can block a functional group in a molecule; the chemical actions are "protection" and "deprotection"

Protein kinase substrate peptide array - An array contains peptides selected from a list of protein kinase substrate proteins or a peptide array made for protein kinase assays

Protein phosphopeptide array - an array contains phosphorylated peptides and array is used for probing phosphoprotein binding domain proteins, such as SH2 proteins.

Radiation strength - The dose of the irradiation, measured as a function of irradiation time and irradiation power.

Reaction sites, array sites, assay sites - used interchangeably describing the nature and activities of sites of an array

Regular building block - Oligonucleotide or peptide syntheses use well established DMT-chemistry or Boc or Fmoc chemistry. These syntheses use standard monomers, which are referred as regular building blocks. Regular building block also implies their use in synthesis cycles typically undergoing deprotection and protein steps.

SNP, Chip-on-Chip, CGH - nucleic acid analysis and profiling related terminologies SNP: single nucleotide polymorphism; Chip-on-Chip: chromatin immunoprecipitation analyzed by chip (microarray); CGH: comparative genomics hybridization.

Solid support - Synthetic media as contrast to gas or solution phases. Solid support include silicon dioxide, inorganic materials, organic polymers and co-polymers such as polystyrene, resin or beads, grafted polystyrene-polyethyleneglycol resin or beads.

Spotting, printing, stamping, spraying - These terms referrer to the mode of liquid handling.

Surface - The side/area facing outwards for a planner plate, bead. or a solid object. A surface is accessible for synthesis or assay

Surface density, dilution, concentration. density-variation sites - Surface density is measured by number of molecules on a unit surface area. An increase in surface density is "concentration" and reverse is "dilution". Array sites which have different surface densities

Synthesis cycle, synthesis steps - reaction steps repeated in oligonucleotide or peptide synthesis. Each synthesis cycles is comprised of several synthesis steps.

Tag, tagging - An add-on portion of a molecule, protein, or tissue or cell samples. There are affinity tags such as flag peptide for antiflag antibody binding, fluorescence dye tag for labeling and detection of molecules and for cellular component visualization.

Terminator, multiplier - these are molecules which terminate further reaction when incorporated into the molecule synthesized or which increase the active site when incorporated into the molecule synthesized. Terminator may or may not be irreversible. Multipliers may be dendrimers of di, tri, or more dentate branches.

TFMSA - Chemical name: trifluoromethanesulfonic acid

The following description of examples is included to demonstrate embodiments of the invention. It should be appreciated by those of skill in the art that the technique disclosed in the examples are representative techniques discovered by the inventor to function well in the practice of the invention.

Described herein are methods for making and use of an array device with improved capability and capacity in terms of the variety and the quantities of the molecules synthesized. An array comprises a plurality of discrete reaction sites which will be used for synthesis of molecules such as oligonucleotides, peptides, carbohydrates, their analogues, or other types of organic molecules. The synthesized molecules at a reaction site are distributed at a certain density which is measured by how many molecules contained in a unit area. In one exemplary of linear peptides which are separated by 40 angstrom apart, there on average will be fmol amount of molecules synthesized on a 50x50 um^2 array site. Each array site will require on average nanoliter or less assay solution for the experiment. When compared with 96-, 384-, or 1,536-well titer plates, the arrays described herein will use at least 1,000 times less assay solution on a per assay (site) basis. Since increasingly assays of genome- or proteome-wide are run in tens of thousands and in hundreds of thousands, these experiments will consume too large of reagents and solutions to make it impractical to carry out high throughput, large scale experiments in many laboratories. There will be thousand liters of assay solutions used and the cost of purchasing, storing, and disposing them would be prohibitively high. Therefore, significant reduction in assay material will remove the barriers for a wide-spread application of genome- and proteome-wide experiments. These large scale experiments will provide invaluable information for understand human biology and advancing biomedical sciences.

It would be highly desirable to perform the experiments now run on 96-, 384-, or 1,536-well titer plates in an array format. One common application of the microwell titer plates is to place substrate molecules such as peptides in a series of known, different concentrations into a set of wells on plate and to which a protein binding solution and then a detection antibody solution are added. By reading the detection signals which are often fluorescence, luminescence or colorimetric signals, the measurements are obtained for deriving biophysical parameters such as binding/dissociation constants of protein- or antibody-substrate systems such as enzyme-linked immunosorbent assay or ELISA, enzyme-substrate activity detection, time dependence parameters of the various systems containing proteins, nucleic acids, carbohydrates, lipids, and/or a variety of small molecules. In enzymatic assays, the density variation sites containing substrate molecules interacting with enzymes to give data sets which reflect product formation as a function of the concentration (density) of the substrate molecules. The experiments can be recorded at various time points. These data can be used to derive enzymatic reaction parameters, such as maximum velocity of the reaction (Vₘₐₓ) and Michaelis constant (K_{M}). The basics of the above description can be found in numerous literature articles and textbooks such as Physical Chemistry.

Accordingly, described herein are synthesis method for controlling surface molecule densities. Specifically the present disclosure reveals methods for preparing arrays of molecules of constant and varied surface densities; different types of surfaces are suitable for the synthesis. The application of the synthesis include generating high density titer plates to allow simultaneously performing small volume assays of nucleic acids, proteins, and other types of molecules quantitatively which by convention are performed using larger volumes of solutions in micro-well plates and sometimes in single reaction tubes. Quantiative analysis provides multiple data points correlated by quantitative relationships and thus the format of the analysis provides more reliable information. The outcome should be suitable for crucial assays directly related to human health, such as clinical diagnosis, prognosis, monitoring of patient conditions, genotyping of individuals, and the applications of the types.

The synthesis methods described herein are based on solid surface containing a plurality of reaction sites or array surfaces and these sites are suitable for in situ parallel synthesis. The surfaces for parallel synthesis may be glass, silicon dioxide, polymer, such as polystyrene, treated polypropylene, grafted polystyrene-polyethylene glycol, PDMS

(polydimethylsiloxane) and its modifiers, or other materials known resistant to chemical reagents, such as methylene chloride or dimethylformamide. For array synthesis intended for on-array surface applications, the surface will also not to produce detection interference, such as absorption or emission background signals which are sufficiently strong to submerge weak signals of the analyte signals. The surface may be planar or porous, may or may not contain coating film patterns which define the array sites, may or may not contain fabrication patterns which define the array sites. The surface may contain dividers or barriers so that subsets of array sites are present which will allow differential treatment of the subset of array sites during and after synthesis. To each kind of surfaces, a suitable parallel synthesis strategy will be selected based on the array synthesis technologies known by those skilled in the art.

Described herein, the array synthesis will be carried out on a bead-loaded surface, wherein bead-loading means immobilization of beads through chemical bond formation between the array surface and the bead on which in situ parallel synthesis of molecular arrays will be carried out

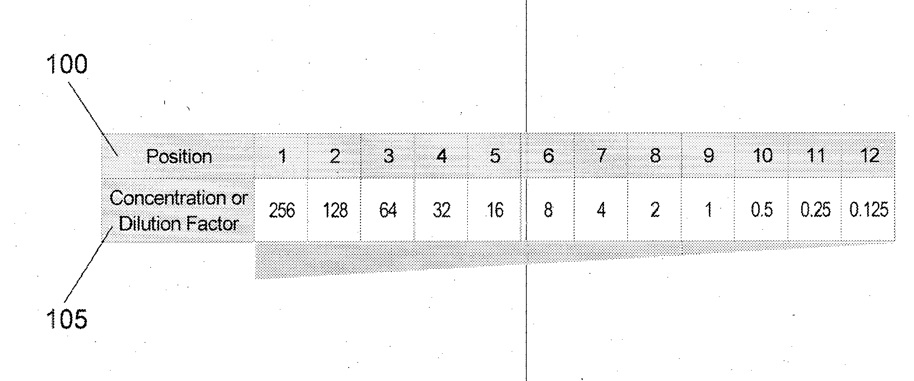

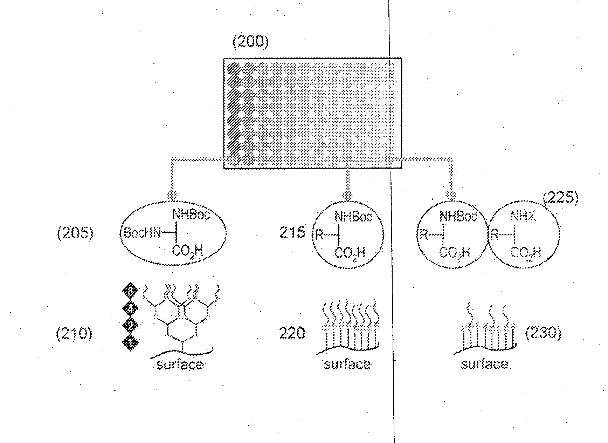

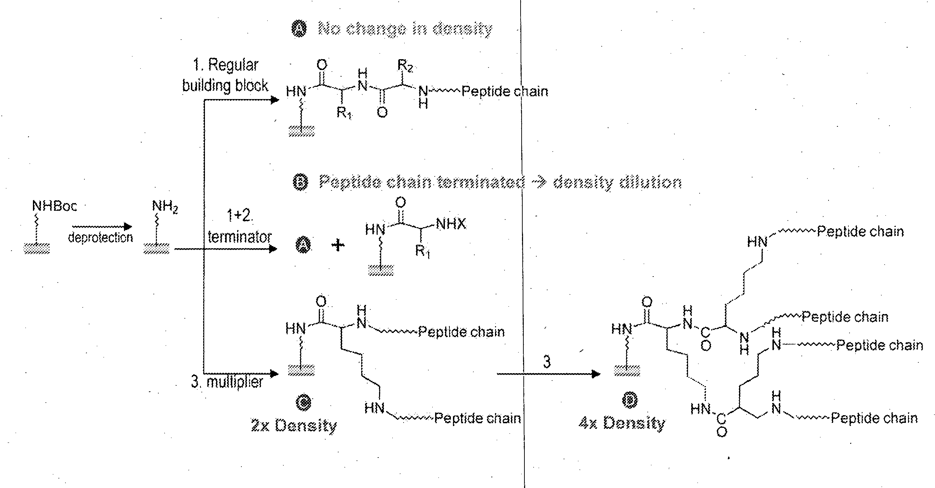

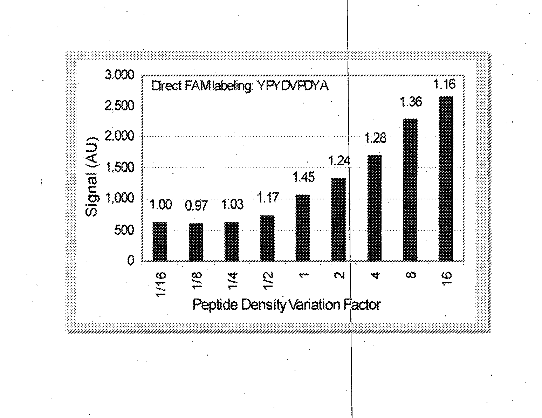

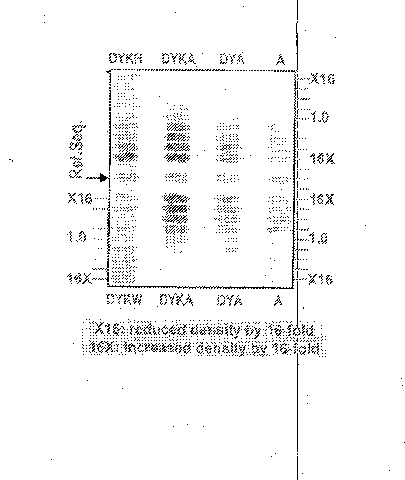

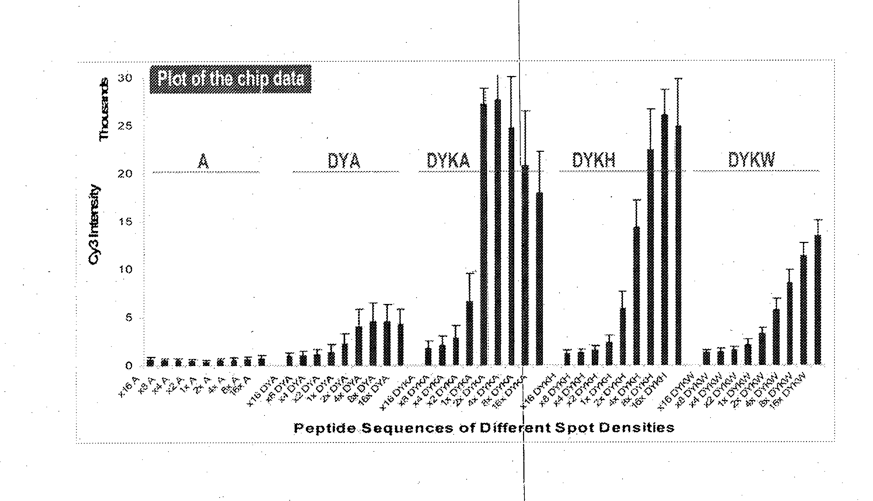

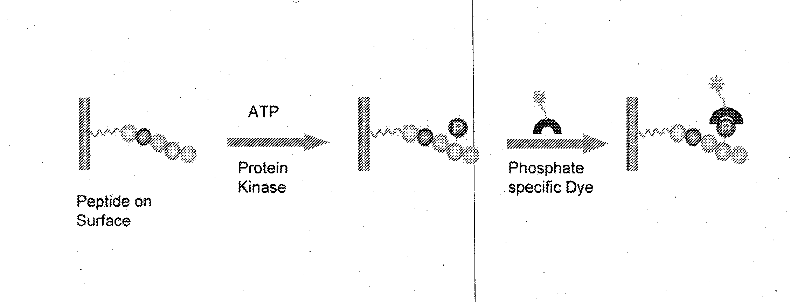

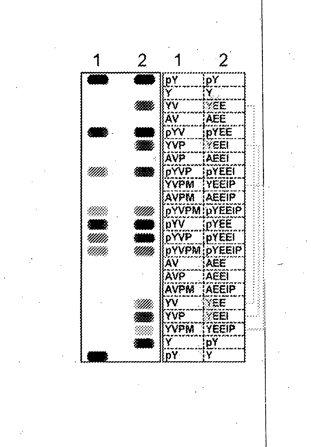

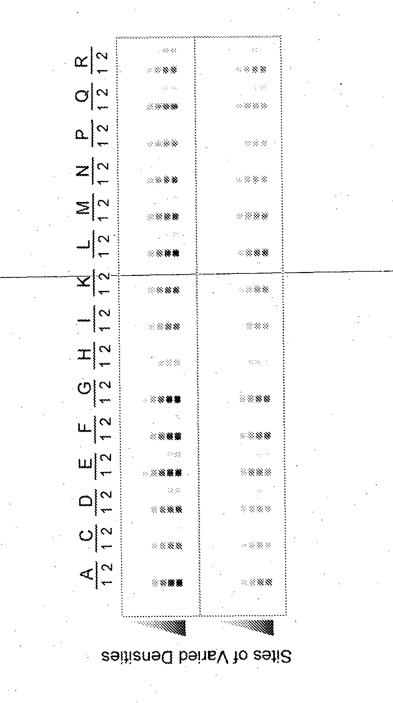

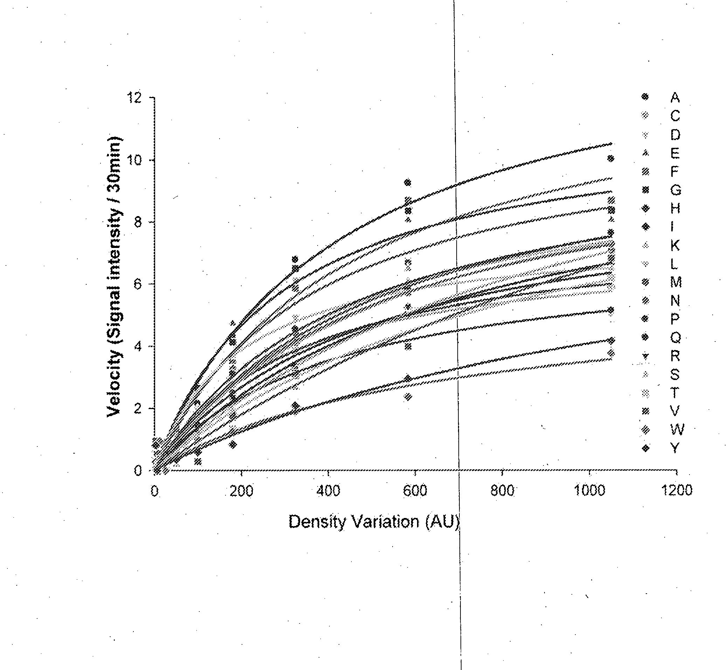

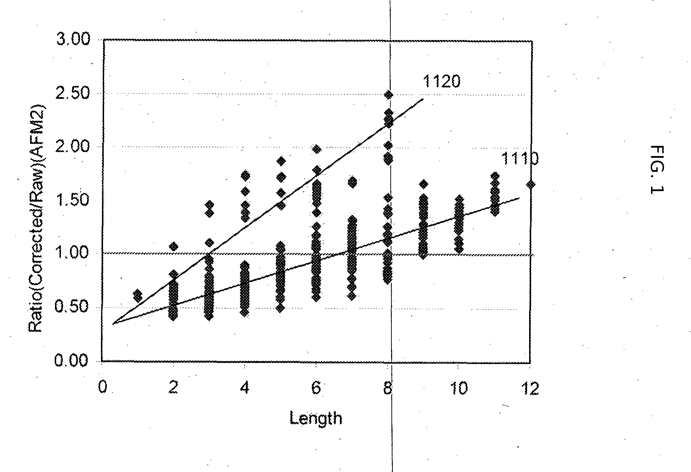

In an exemplary embodiment, FIG. 1 illustrates the concept of an array of molecules at different reaction sites with controlled varied densities. The first row (100) indicates the position of the density variation sites and the second row (105) indicates the density variation factor, i.e., by how many fold with 1 being the original density when no dilution or concentration of synthesis is used. The size of the factor will be depending on the reaction reagents used. FIG. 1 shows the factor equals two for each adjacent sites and a total of 2,048 fold of change in molecular density for 12 sites. In fact, dilution and concentration can be used in combination to create continuous ratio variations at various reaction sites. On an array of 3,849 sites, 40 conventional microtiter plates of 96-wells will be simultaneously created. This miniaturized assay device will provide at least 40-times faster throughput but consumes only a fraction of the reagents and solutions and requires only a fraction of human resources. High density arrays have even greater potentials, an array containing 100, 200, 400, 800, 10,000, 100,000 or more conventional microtiter plates of 96-wells will be possible.

It is clear to those of skilled in the art that surface molecular density measures number of molecules in a unit area and thus is a two dimensional parameter, while molecular concentration measures molecules in a unit space and thus is a three dimensional parameter. However, these two parameters are comparably if one considers the spatial separation distance defined by a center to center distance between two molecules, the relative surface density of two surface sites is representative of relative concentrations of two samples in a container. Therefore, the significance of a density-variation array is in its applicability for quantitative measurements of the interactions between analyte and surface molecules. In the presence of an internal calibration, known sequence and the related density-variation data points, the array measurements of the unknown analyte can be used to derive parameters close to conventional biochemical measurements.

The molecular density-variation sites may be created by several methods. One approach is to allow partial activation or reaction at the surface between the surface factional groups and the added reactive molecules. In an exemplary embodiment, the surface functional groups are protected amino groups. When the protecting group is light labile, a full dose of light irradiation will completely remove the protecting group; a partial dose of light will only deprotect a fraction of the surface amino group. The percentage of the deprotection will be proportional to the strength of the light dose. Alternatively, the surface protecting group is acid or base sensitive, such as the acid-labile moieties of DMT or Boc and the base-labile moiety of Fmoc for the amino group. The percentage of the deprotection will be proportional to the strength of the light dose which induces acid or base formation in the presence of photogenerated acid precursor or photogenerated base precursor as shown in the work of Pellois et at (2001) and LeProust et al. (2001) (Pellois. J. P. Wang, W. and Gao, X. (2000) Peptide synthesis based on t∼Boc chemistry and solution photogenerated acids. J. Comb. Chem. 2, 355∼360, Leproust, E., Zhang, H., Yu, P., Zhou, X., Gao, X. (2001) Characterization of oligodeoxyribonucleotide synthesis on glass plates. Nucleic Acids Res, 29, 2171-2180). However, the light irradiation controlled methods cannot be used to increase the surface molecular density.

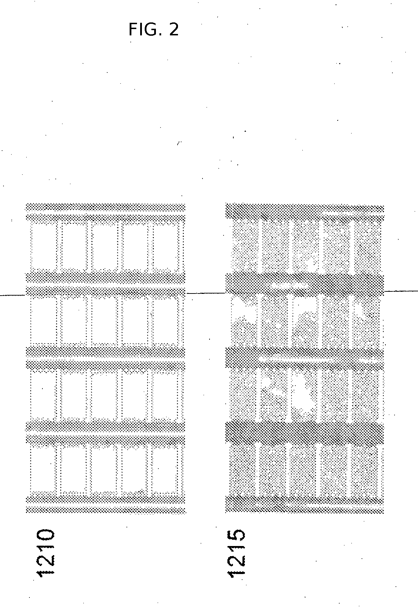

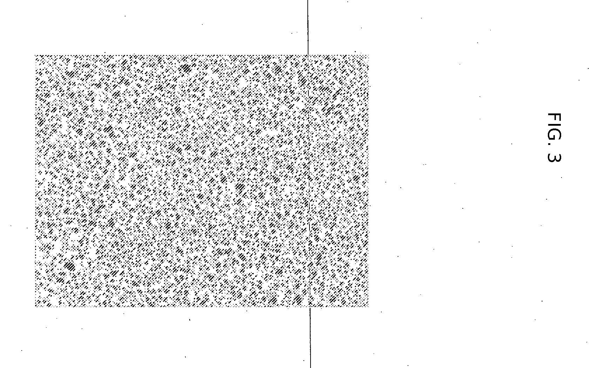

Alternatively, chemical synthesis method may achieve increasing, no change, or decreasing molecular densities on array sites by adding appropriate reagents (FIG. 2). In one example, the method is to react a multiplier molecule (205) with the surface functional group to increase the molecular density at a predetermined site (200). In theory, the surface density will increase by a factor of two at each step of coupling of a bidentate dendrimer and at the end of four cycles of synthesis, there should be eight times more molecules on the same surface area (210). In another example, the method is to react a regular monomer (215) with surface functional group to keep the molecular density at a constant (220). In yet another example, the method is to react a mixture containing terminator (225) to reduce the molecular density at a predeterimined site (230). The relative concentrations of the reagents used may be according to FIG. 1., second row of the table (105) wherein #9 spot is according to FIG. 2 (215), such as a Boc- or Fmoc-protected amino acid, and the protected amino acid couples to surface amino group and the coupled amino acid will undergo subsequent reaction in next synthesis cycle. According to FIG. 1, spots #8 through #1 will be synthesized in eight steps and a doubler such as N,N-(Boc)2LysCO2H or N,N-(Fmoc)2LysCO2H or N,N-(NPPOC)2LysCO2H (wherein NPPOC is 2-(2-nitrophenyl)propoxycarbonyl, a photolabile group) will be incorporated. According to FIG. 1., steps #10 to #12 will be synthesized in three steps and a mixture of 1:1 regular monomer such as a protect amino acid, such as Boc-Ala, Fmoc-Ala, or NPPOC-Ala, and terminator amino acid, such as Ac-Ala, will be incorporated. The mixture reagent will block 50% of the surface molecules which couple with the terminator amino acid at each synthesis step. The sequential steps of preparation of density-variation sites in the three (density increase, no change, or decrease) are illustrated in FIG. 3.

In general the amounts of the molecules of each type, or several types on different sites, or several types on the same site, made by in situ synthesis on miniaturized devices, can be increased by several folds or more. This is not limited to the methods described above but many coupling and chain length extension reactions can be used. A bidendate dendrimer will increase the density of the molecules synthesized by a factor of two for each cycle of synthesis, similar to that of the exponential growth of the polymer chain amplification. The amplification factor for a n-branching point molecule will be n^{k}, where k is the number of reaction cycles,

It is clear to those skilled in the art that the actually surface density of the density-variation factor tends to be less than that of theoretical due to deficiency in synthesis yield. The factors that influence the synthesis on density-variation sites also include surface packing effect Especially when surface molecule density increases, the space that allows molecules aligning in parallel decreases. To certain degree and for certain molecular systems, there may be unfavorable steric interactions or surface energies of positive or negative charge, which would reduce or inhibit molecular density infinitively increase. One must consider the less than perfect synthesis yield cost by these and other unfavorable factors when consider the final surface density of an array site.

The density-variation sites may be organized like a 96-well titer plate and the design of the array layout makes careful considerations to assign each sequence and each density-variation site a predetermined position/address on the array surface. The specific density of an array site or its relative density to other array sites needs not to be identical by using in situ parallel synthesis method. On different reaction sites, different "amplification" factors will be possible by synthesizing different numbers of the branching dendrimers in the growing chains. This will generate molecules in different stoichiometric ratios. The molecules synthesized are not limited to peptides but DNA and RNA oligonucleotides, carbohydrates, and other categories of polymers and combinatorial synthesized molecules.

The method of density-variation array synthesis begins with the design of the density variation patterns and synthesis steps followed by organizing the array layout for the sequences to be synthesized. At least a set of density-variation data for at least one sequence will be collected from the array. The final array layout file will be used to direct the synthesis of the array on synthesis instrument

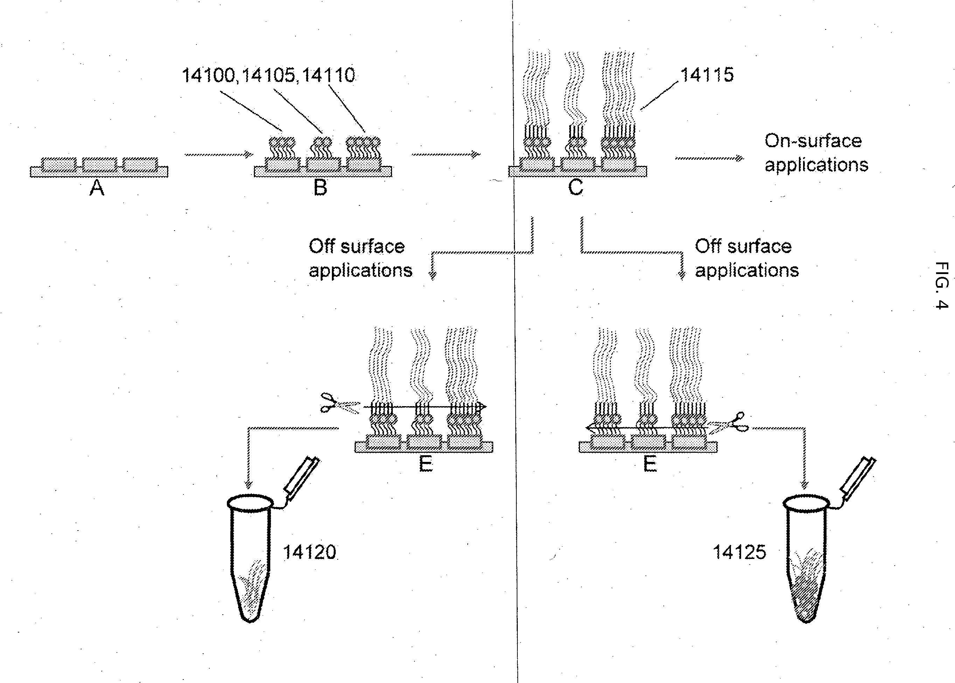

In one example of peptide array synthesis, human influenza virus hemagglutinin (HA) epitope peptide YPYDVPDYA (SEQ ID NO: 4) was synthesized at density-variation sites. These peptides at different array sites were coupled with fluorescence molecule in the synthesis, giving arise to detection signal which is proportional to the density of the molecules per varied density site (FIG. 4). The density varied in synthesis from 16-fold dilution (1/16) to 16-fold concentration (16), to give sites of 1/16, 1/8 1/4, 1/2, 1, 2, 4, 8, 16 in density. The plot shows the observed ratio of the two varied density sites for each 2-fold dilution or concentration. The anti-HA antibody (100 g/mL) in 1x PBS buffer was applied to the peptide array surface and the binding data on the density variation sites revealed that the observed ratio of the two varied density sites for each 2-fold dilution or concentration. The sigmoid plot illustrated that peptides of varied densities are equivalent to concentration variation sites, producing binding curves. This binding curve produces binding constant in the order of 10^(-7) M by curve fitting based on a classic isotherm binding model, consistent with high affinity antibody binding on the epitope peptide array. The derived binding constant may be used as a calibration value since the binding constant of anti-HA antibody to its epitope is known to be in the order of 10^(-8) M. The discrepancy for the measurements from an array or a solution will be compensated.

Further binding experiments illustrates simultaneous measurements of multiple binding curves on a peptide chip containing peptide ligand sequences. The binding experiment used anti HA antibody (Roche) and the concentrations of anti HA (antibody) varied from 0.1 to 60.000 ng/ml. The binding was in TBS buffer, pH 6.8, one or more hours at 4 °C. The signal intensities were from a secondary antibody conjugated with a fluorescence dye (cy3 or cy5) which recognize anti Ha bound to peptide sequences. Signal intensities acquired at different scanning gains of a laser scanner were scaled according to a calibration curve of the instrument. Curve fitting used Origin (Origin Lab) to produce binding constants.

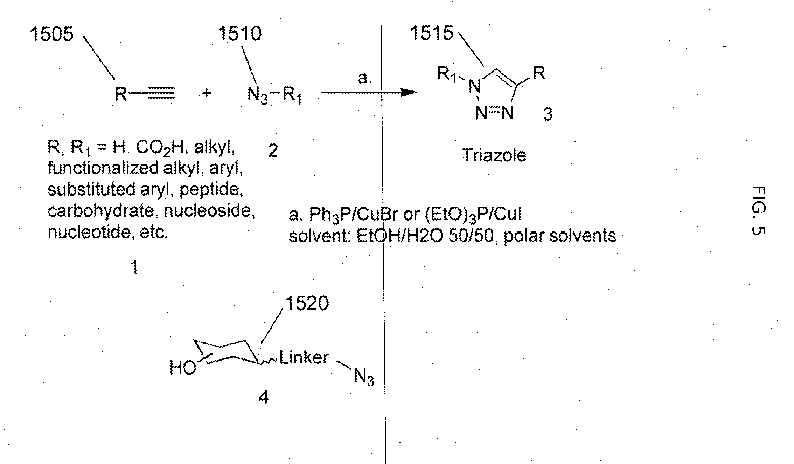

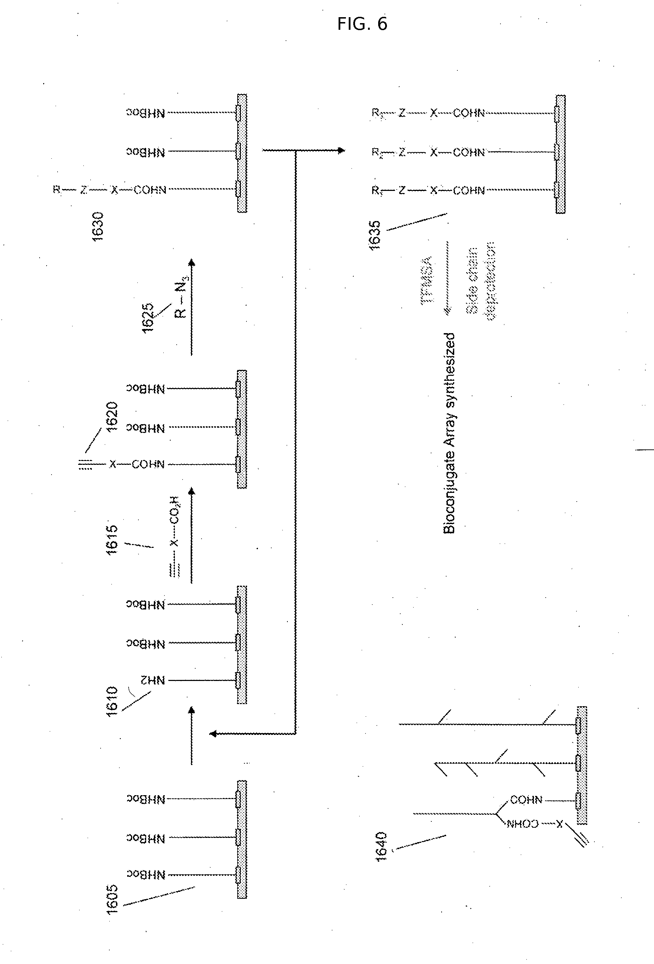

In one example wherein the synthesis can be monitored by directly labeling with a fluorescent molecule as the last step of synthesis or by a fluorescent dye tagged binding molecules to the molecules synthesized on surface. FIG. 5 is a fluorescence image of a peptide array containing antibody binding sequences on density-variation sites. The synthesis of the density-variation sites varied from using 16-fold dilution (x16) in synthesis cycle 1 to 16-fold concentration (16x) in synthesis cycle 9 to give a series of nine spots for each of the sequences: DYKH, DYKW, DYKA, DYA, and a single amino acid A. The relative densities of these array sites are shown in fluorescence signal intensity gradient. The fluorescent signal observed for molecule A. Specifically, the fluorescence intensities were from binding of cy3-anti-flag peptide antibody (AFM2) to the peptides on array surface. Peptide sequences are represented by single letter code of amino acids (A: alanine, D: aspartic acid, H: histidine, K: lysine, W: tryptophan, Y: tyrosine) (FIG. 6). The synthesis began with a surface containing Boc-amino groups; selected sites are deprotected to give free amino groups which were coupled with a mixture of 1:16 of regular amino acid and chain terminator amino acid. The deprotection reaction was repeated on a second set of sites to give free amino groups which were coupled with a mixture of 1:8 of regular amino acid and chain terminator amino acid. These deprotection and coupling steps were repeated for the mixture of 1:4 and 1:2 of regular amino acid and chain terminator amino acid. The subsequent deprotection was performed and coupling used regular amino acid. The deprotection reaction was repeated again to give free amino groups which were coupled with N,N'-(Boc)2Lysine. The deprotection and coupling with N,N'-(Boc)2 were repeated three more times. This nine steps of synthesis produced molecular sites of varied densities as shown above. The subsequent peptide synthesis was carried out as described previously. In one example, the Boc-group was removed from a predetermined set of varied density sites to give free amino groups which were subsequently coupled with Boc-alanine. The repeated synthesis cycles of selective deprotection at predetermined sites and coupling reactions produced peptide array containing peptides at density varied sites. The binding curbe plot (FIG. 6) further shows that peptides of varied densities are equivalent to concentration variation sites, producing different binding intensities upon binding to antibody. Peptide array can be used as binding curve titration plate for monitoring binding and comparative studies of the antibody binding affinities to a plurality of epitope peptides.

Antibodies are known to cross binding to peptides which induce non-specific binding. This property is not desirable for antibody as purification tag or as therapeutic agent. Peptide high density array offers a broad range of search of non-specific binding of antibody to peptides and provide the sequences of these peptides; further the peptide array offers quantitative measurements (binding curves) of the interactions. These capacities of peptides make it a powerful tool for specific antibody and epitope screening, for reducing false positive or false negative detections. Using binding curves rather than a single binding data point is also an effective way to identify biomarkers which will produce more sensitive and reliable measurements.

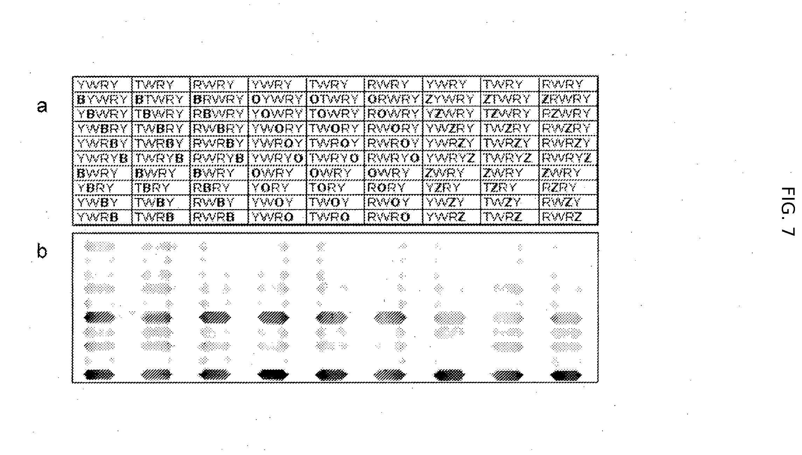

Peptide array containing protein kinase substrate peptides with density-variation sites can be synthesized as described above for epitope peptide array. FIG. 7 illustrates a form of protein kinase assay involving peptide chip and protein kinase enzyme. The peptide sequences on the chip surface are exposed to enzyme and enzymatic reaction solution. The peptides which are substrates of the enzyme are thus phosphorylated. The presence of the phosphate moiety is detected by a staining dye molecule conjugated with a chemical moiety which specifically recognizes the phosphate group. Protein kinase assays have many different forms known to those skilled in the art and thus the peptide chip-based kinase assays are not limited to the form shown.

The present disclosure provides method for universal dye staining of phosphate on the sequences of a density-variation peptide array. Fluorescence mage was obtained applying a phosphate-specific reagent, cy3-Pro-Q (Invitrogen, CA, USA), to peptide chip containing pS and pT substrate phosphopeptide sequences. The observed signals are consistent with the present of pS or pT-containing peptides (phosphopeptides) and the density gradient curves are also observed. The intensities may be calibrated for quantitation of the density of the peptides at individual sites. The phosphate staining reagent may be other compounds used in magnetic resonance imaging and else where as contrast agent in biological samples. This method overcomes the general problem of pS- and pT-peptide detection, since the antibodies for these peptides gave poor binding and show sequence specificity, making their detection difficult. The method may be used for sensitive detection of kinase activities on peptide array and quantitative analysis of kinase activities.

The present disclosure provides method for parallel synthesis of phosphopeptide array containing density-variation sites. According to the chemistry and procedures described In FIG. 1, FIG. 2 and FIG. 3, an array containing density-variation sites is first designed and the proper array surface is functionalized according to the surface density-variation layout file. On this surface, phosphopeptide array will be synthesized using a method available for parallel synthesis of the peptide array, such as the uParaflo method as described by Zhou et al. (2004)¹¹. Alternatively, SPOT method may be used as described by Frank and co-workers^{18 19}.

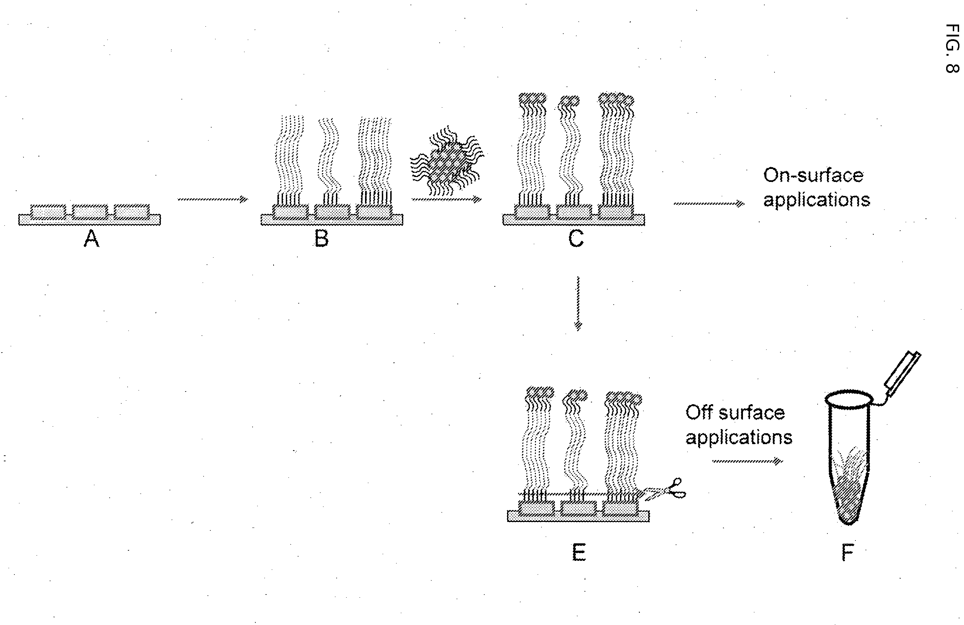

FIG. 8 is a fluorescence image of a protein kinase reaction on pY-phosphopeptide chip. The experiment used Src kinase, p60c-src (Invitrogen). The chip included 23 known kinase substrates and their sequence variants, which vary in length and composition, a number of position and negative control sequences, and a few artificial peptide sequences. For each substrate peptide, at the phosphorylation position. Tyr (Y) in YEEI, for instance, three sequences are present, which are YEEI, pYEEI and AEEI. pY-containing sequences are directly synthesized which are reference sequences for the Y-containing sequences (as the substrate for kinase reactions). There are also background sites which contain no peptides. 8-22 redundancies were present. The peptide sequences shown in the image are YVPM (column 1) and the YEEIP (column 2) related sequences in double replicates. These sequences are given on right of the image. The results show that YEE and YEEI are phosphorylated by the Src kinase used, and comparatively, YEEIP is a substrate of lower reactivity, and YVPM has the lowest reactivity. Kinase reaction conditions and phosphorylation detectdion were: 10x enzyme storage solution (0.5 mg/ml), 50 ul reaction buffer, pH7.5 (50 mM HEPES, 0.1 mM EDTA, and 0.01% Brij 35, 0.1 mg/ml BSA, 0.1% beta-mercaptoethanol, 0.07 mM ATP, 10 mM MgCl₂), 25 °C for 30 min. The array was stained by filling the array with 200 ul cy3-Pro-Q solution (phosphate staining reagent), standing at room temperature for 20 min. The chip was then washed with 2 m! destaining buffer, pH 4.0 (50 mM Na₂CO₃, 20% acetonitrile) as recommended by the vendor. The image was obtained from a regular microarray scanner (Axon GenPix 4000B) and shown using inverted intensities after background subtraction.

The signal intensities were analyzed using the following equation:

Relative phosphorylation efficiency: fₚ = (I_{Y}-I_{A})/(IₚY-I_{A})

For a peptide, 1 is the signal intensity, I_{Y} is for Y-containing sequence which undergoes phosphorylation by kinase, I_{pY} is for the synthetic phosphopeptide, I_{A} is for the sequence containing Y→A substitution. By using the positive controls (pY-sequence) and negative controls (A-sequence or other non-phosphorylation amino acids) to derive relative phosphorylation efficiency, fₚ as fraction or percentage, the kinase reaction results were analyzed quantitatively. The method takes the advantage of addressable peptide array for reducing false positive readout and improves reliability of the data analysis.

A protein kinase substrate peptide array gave a fluorescence image of a PKA assay shown in FIG. 9. Peptide sequences containing phosphorylated serine (pS) residues are displayed by positive signal from cy3-ProQ (phosphate-specific staining agent, Invitrogen). The sequences for each two columns are: 1. RR-X-SL (SEQ ID NO: 17-31) 2. RR-X-AL, X (SEQ ID NO: 31-44) is labeled in the FIG 9. and N-terminus was acetylated. Two redundant data sets are presented. The varied densities ranges from 32-fold dilution to 16-fold concentration of the original synthesis density using the synthesis methods disclosed in this invention. Each peptide sequence was synthesized on these ten varied density sites. The X-S containing sequences are potential substrates for PKA, X-A containing sequences are negative controls, and X-pS containing sequences (now shown) are positive controls. PKA reaction conditions: PKA kinase reaction buffer (50 mM Tris, 10 mM MgCl₂, 2 mM ATP, 0.1% BSA, pH 7.5) 1 mL was flow through the chip for 20 min. This solution was replaced by 2.500U cAMP-dependent Protein Kinase (PKA catalytic subunit (New England BioLabs) in fresh 50 µL of kinase reaction buffer and reaction continued at 30°C for 30 min or longer time. The reaction was terminated with 4 mL Milli-Q H₂O washing. Detection: 200 µL Pro-Q staining solution (Invitrogen) was circulated through the chip for 20 min and followed by 4 mL Milli-Q H₂O washing. Finally the peptide chip was washed with 4 mL destaining buffer (100 mM NaHCO₃/CH₃CO₂H, pH 4.5, 20% acetonitrile) at room temperature before image scanning.

The corresponding plots of the above density-variabon data from a PKA reaction for 20 RR-X-SL peptide sequences are shown in FIG. 10. Each of these sequences was synthesized on varied density sites (FIG. 9). The plot is the measured fluorescence signal/reaction time (30 min) *versus* varied densities in arbitrary units (AU). Separately, at each density point of a peptide, a plot of velocity (v) *versus* time of the reaction is determined. The initial reaction velocity (vₒ) is derived from the slope of this plot in the region where the function is linear, for each peptide at each density variation point. A similar plot to above but velocity is vₒ produces a curve from which maximum velocity of the reaction (Vₘₐₓ) and the K_{M} are determined as described in textbooks of Biochemistry (e.g. Biochemistry, 5th Ed. Berg, J. M., Tymoczko, J. L., Stryer, L. (2002) W. H. Freeman and Company, New York, pp. 200-222). These kinetic parameters are also derived from the intercepts of the x- (= -1/K_{M}) and the y-axis (= 1/Vₘₐₓ) of the Lineweaver-Burke double reciprocal plot

The applications of protein kinase substrate arrays are related to several areas. The peptide array can be a kinase detection tool for identify a kinase in a complex biological sample or identification of a novel protein; the peptide array can also be of use for profiling of cellular kinases. Due to the central role of protein kinases, these applications will have significant impact on not only research but also disease diagnosis, therapeutic target identification, inhibitor screening, for example.

The present disclosure provides a method for improving the quality of quantitative measurements of array experiments. The following data processing involves procedures for removing noise from binding signals. In particular this noise is from the differences in synthesis qualities. For arrays of diverse molecular moieties, the measured signals become increasingly inaccurate due to unknown synthesis qualities and it is impractical to optimize the synthesis to its perfection. Therefore, it is useful if the synthesis quality parameter can be considered in array data analysis. The method according to the present invention is in the last step synthesis on chip to use 1% fluorescein direct coupling of the surface functional group in the molecules synthesized, such as the terminal amino group in peptides. Following the synthesis, the fluorescein was activated and image was acquired. The fluorescence signal was I_{F} and the background signal was I_{bg1}. This peptide array contained anti flag peptide epitope and various peptides (total 414 sequences) of 2- to 12-mer in length with at least four replicates (#050013). The side chains of the peptides were then deprotected using the TFMSA protocol for Boc-chemistry synthesis. The array was washed with water and binding TBS buffer. The binding experiment used100 ng/ml anti flag M2 (AFM2) at 4 °C, TBS buffer, pH 7.5, 1 hour. The binding was detected through cy5-IgG staining (100 ng/ml) at 4 °C, TBS buffer, pH 7.5, 30 min. The cy5 signal of the antibody binding was recorded using a microarray scanner (Axon GenPix 4000B). The data sets contained cy5 signal (I_{b}), background signal (I_{bg2}), and from previous image, I_{F}. From these data, raw binding intensities for binding were determined by I_{b} =I_{b} - I_{bg2} and direct fluorescein labeling intensities were determined by ΔI_{F} = I_{F} - I_{bg1}. The ratio R_{bF} (= I_{b}/I_{F}) gives binding molecules per unit fluorescence reading of the synthesis or this is called corrected binding data. Next, R_{bF} and Δl_{b} are balanced in overall intensity and the correction fact is derived by comparing the two binding intensities (corrected/raw) and these are plotted above for the 414 sequences in Fig. 11. For each length, two groups of correction factors are observed: those along the line 1110 and those along the line 1120. The slope of line 1110 is greater than line 1120. The sequences of the line 1110 group are those of greater correction factors when the sequences of the same lengths are compared. The greater correction factor in this case is due to lower synthesis yields in certain amino acids. For each line, line 1110 or line 1120, the trend is the longer the sequences, the greater the correction factors. This is consistent with the length effect in synthesis efficiencies. When correction factor is less than one, such as those for shorter sequences, the actual assay intensity is less than without the correction or vise versa.

The method of binding signal correction for synthesis deficiencies has a number of benefits. Normally, one assumes the higher the binding intensity, the stronger the binding without taking into consideration of the difference in chain length and/or sequence compositions. However, in one example of peptide synthesis, the synthesis yield is not 100% and 20 amino acids give different synthesis yield as well. These deficiencies in synthesis may lead to erroneous conclusions in analyzing the binding data. For instance, if the stepwise synthesis yield is 95.0%, a 3-mer sequences will be 1.6-fold of the 12-mer sequences. When the binding data is evaluated, this difference in synthesis yield should be considered. The direct fluorescence labeling provide a means for quantitation of the amount of the synthesized molecules, allowing the length effect in synthesis to be deducted. By the same token, the difference in synthesis yield by different amino acids is also deducted. The assay data are evaluated based on signal/signal(molecules on surface).

One should note that fluorescence or other types of labeling molecules may affect the subsequent assays due to higher background signals. This can be overcome by allowing low percentage of labels or attaching the label through a cleavable linker. After the signals for synthesis are acquired, the labels are removed without affecting the subsequent applications. A removable linker between the sequence synthesized on surface and a label may be base labile, such as ester linkage or 1.2-diol moiety-containing linkers, or acid labile, such as those cleaved to generate carboxamide moieties, reductive cleavable, such as the disulfide linker, or light labile, or enzymatically cleavable, or as those well-known by those skilled in the art. It is important to select label and conditions so that the signal reading for synthesis is linearly proportional to the amount of sequences on surface.

The above discussions should be generally applicable to the signal analysis of the array data from enzymatic reactions, hybridization, and various array assays. For different array systems such as peptides, modified peptides, phosphopeptides, dye-tagged peptides, oligonucleotides, carbohydrates, the ratio parameters will be different and adjusted method for different synthesis, for different types of molecules, and for different direct labeling signal used.

The methods of the present dislcosure as discussed above for density-variation array design, array synthesis, array quality control, and array data analysis are not limited to peptide arrays. The general procedures and steps are suitable for making and use of DNA/RNA oligonucleotides arrays, carbohydrate arrays, and a diverse family of arrays containing chemical modifications of these groups of molecules.

The present disclosure provides methods for effective peptide array synthesis, Parallel synthesis using conventional chemistry that is activated by photogenerated reagents, such as photogenerated acids or bases, has been demonstrated for synthesis of DNA/RNA and peptide arrays, and arrays of modified DNA/RNA and peptides. In one example wherein peptide array is synthesized by deprotection using a photogeneraed acid precursor, triaryl sulfonium salt The deprotection time for removal the N-Boc group on the terminal chain under this condition is more than 10 minutes, or more than 12 minutes for more complete deprotection of the amino group. This long time required for peptide array synthesis is problematic. The comparison of the deprotection efficiency of sulfonium salt and iodium salt revealed that the latter took only less than one minute or less than 30 sec. to completely remove the Boc-protecting group, representing a significant reduction of the peptide array synthesis time. The new reagent suitable for peptide array synthesis becomes more important for density-variation peptide arrays due to increased synthesis steps are required.

One experiment compared two sets of deprotection conditions: Reaction A used iodenium salt in the presence of 2-isopropylthioxanthone in CH₂Cl₂ in the deprotection step for removal of the acid labile Boc group of amino groups on surface. Reaction B used a different photogenerated add precursor. The deprotection used different irradiation times (from 1 sec to 10 min light irradiation). The fluorescence moiety was coupled to the peptides on surface after all the deprotection steps were completed. The darker the spot, the stronger the intensities, the more effective the deprotection conditions. Reaction A is more effective in 20 sec to one minute than reaction B in 7 or more minutes. The deprotection of the Boc-group can be achieved on time scale of seconds. The peptide arrays synthesized using iodium salt are validated with standard binding experiments using anti-flag antibody AFM2 and experiments produced comparable results to the existing

Reducing the synthesis cycle time is critical for practical use of in situ parallel synthesis of peptides. The synthesis cycle time is a function of synthesis instrument and thus improvement in instrumentation contributes to shortening of the synthesis time as well. These include measures of stronger light source (without sacrificing optical resolution), delivering heat to the chip, microwave application to the chip synthesis area, and other methods well-known for acceleration of the rate of organic reactions.

The present disclosure provides method for significantly increasing the capacity of an array as a synthesizer. A high density array surface herein is defined as a high number of molecules synthesized per unit area, and thus a high capacity array is comprised of high density array surfaces. One method is to load and immobilize porous synthetic media to a support surface such as a planner glass as used at the present time as the surface of array synthesis. The porous synthetic media such as polystyrene beads (Pierce, USA), TentaGel beads (Rapp-Polymere, Germany), and controlled porous glass beads (CPG or LCAA-CPG, Sigma, USA), can generate at least 10 times more materials and 100, or 1,000 times or more can also expected. In one embodiment of the present invention, 10 um TentaGel beads are loaded into a microfluidic array chip, which was described in Zhou at al. 2004 (Zhou, X., Cal, S., Hong, A., Yu, P., Sheng, N., Srivannavit, O., Yang. Q., Muranjan, S., Roullard, J. M., Xia, Y., Zhang, X., Xiang, Q., Ganesh, R., Zhu, Q., Makejko, A., Gulari, E., and Gao, X. (2004) Microfluidic picoarray synthesis of oligodeoxynucleotides and simultaneously assembling of multiple DNA sequences. Nucleic Acids Res. 32, 5409-5417). The surface of the chip was derivatized with an amino propylsilane linker and the amino group was then reacted with succinnyl anhride. The beads (TentaGel M NH2, 10 um) were from Rapp-Polymere (Germany). The beads were loaded into the array sites (microfluidic chambers) (FIG. 12, where in 1210 has empty reaction cells and 1215 is filled with the beads). The beads react with the amino group on array surface and increase the surface synthesis capacity dramatically. TentaGel beads are wildly used as solid support for synthesis of oligonucleotides and peptides.

In an example shown in FIG. 13 wherein oligonucleotides are synthesized on a glass plate loaded with 10 um TentaGel beads. The glass surface was derivatized as described by Zhou et al. (2001 (Zhou, X., Cai, S., Hong, A.. Yu, P., Sheng, N., Srivannavit, O., Yong, Q., Muranjan. S., Rouilard, J. M., Xia, Y., Zhang, X., Xiang, Q., Ganesh, R., Zhu, Q., Makejko, A., Gulari, E., and Gao, X. (2004) Microfluidic picoarray synthesis of oligodeoxynucleotides and simultaneously assembling of multiple DNA sequences. Nucleic Acids Res. 32, 5409-5417) and was placed in a 1 umol DNA synthesis column. The column was connected to a DNA synthesizer (Expedite 8909, Applied Biosystems) and an oligonucleotide 15-mer was synthesized using the standard DMT chemistry and a standard synthesis protocol provided by the vendor. After synthesis, the protecting groups of the nucleotide bases were removed. Four tests were run to validate the synthesis: (a) direct labeling of the oligos synthesized by fluorescein and the detection of fluorescence signals as described in (Leproust, E., Zhang, H., Yu, P., Zhou, X., Gao, X. (2001) Characterization of oligodeoxyribonucleotide synthesis on glass plates, Nucleic Acids Res. 29, 2171-2180); (b) UV absorption scan of the cleaved products from the surface and a positive reading at 260 nm; the glass plate loaded with TentaGel beads produced at least 1,000 times more oligos compared to that produced from the glass surface: (c) HPLC analyzed the oligo produced on bead-loaded glass plates, the observed retention time was 16 min, in agreement with a standard sample (HPLC column (RC): C18, 8x10 10 µ: flow rate: 1.5 mL/min, UV wavelength monitored: 200-600 nm; solvent A is CH3CN, elution solution B is 0.05M TEAA buffer plus 1% CH3CN, gradient 5% A 2 min, 5-35% A, 20 min); (d) hybridization on bead-loaded glass plate. The hybridization complementary strand of the oligo synthesized on bead-laaded plate was synthesized on CPG support and 500 uL solution of 0.5 uM hybridization cy3-labeled oligo was used. For comparison, a bead-loaded glass without synthesis of oligos was treated by the same procedure as that used for bead-loaded glass plate with synthesis of oligos. The glass plates were imaged under epifluorescence microscope and stronger fluorescence signal was observed from the glass plate containing synthetic oligos.

In the method of the present invention described above, oligonucleotides synthesized on the bead-loaded glass plate were removed from surface for off-array surface applications (FIG. 14). It is not necessary that all array sites to have the same surface density or capacity (14100, 14105, 14110). The linkages between the surface and the beads and between the bead and the oligonucleotide are stable under synthesis and deprotection conditions of oligonucleotides as described in (Gao, X. and Yu, P. "Novel reagents compounds and methods of making and using the same". PCT International Publication WO 2005/000859). One example of such a linker is the amide bond linked alkyl chain, For on-array surface applications (14115), there are a large fold more surface molecules at each array site; this will lead to more sensitive detection. The dynamic range of the detection may also increase since while the low detection limit is about the same as that of conventional DNA oligonucleotide arrays, the upper detection limit is much higher. For off-array surface applications (14,120, 14,125), the cleavage bond is designed so that the cleavage is to occur to release either products without the bead attached (14,120) or with bead attached (14,125). Although the cleavage can occur during the final deprotection stage after the molecule is synthesized, it is desirable to cleave the product synthesized at the last step succeeding the final deprotection steps. The subject of linker use in oligonucleotide synthesis is discussed in detail (Gao, X. and Yu, P. "Novel reagents compounds and methods of making and using the same". PCT International Publication WO 2005/000859).

In the method of the present disclosure, the bead-loaded array capacity is determined by the specific configuration of the loading beads. The example used herein is 10 um TentaGel beads which have sub-picomol per bead functional groups and there are millions beads per gram of bead weight. If the array site is of the size 50x50 square micron, there may be up to hundreds of pool molecules or more than 10°(14) molecules synthesized per array site. Therefore, the bead-loaded glass plate as media of synthesis produces, in one example, wherein nmol of molecules synthesized (FIG. 13). Importantly, in an array synthesizer, hundreds, thousands, up to millions of different molecules are simultaneously synthesized; while it would require many batches of synthesis is required for the prior art of 96- or 384-well synthesis for making up to millions of different molecules. The use of chemical materials by the prior art of synthesis will also be prohibitive for it to be useful in the very large scale applications. It is well-appreciated by those skilled in art that array synthesizer has major advantages over the prior art of micro-well based synthesis and is essential for nano- or smaller-scale bioassay devices running ultra-high throughput genome- or proteome-wide experiments

The method demonstrated herein are not limited to the materials of glass plate and TentaGel beads, not limited to the 10 um beads, not limited to spherical supporting for synthesis. Other sources of materials of different chemical (such as PDMS, polystyrene) or physical (such as total plat, having etched, layered, multi-sided) configurations for high capacity synthesis appreciated for those well-known in the art may also be choices as well for intended array synthesis. The surface of the array may be modified to contain wells, holes, shallow or deep channels, or other physical modifications so that the surface of the array is divided into subsets of areas, or add other feature to facilitate subsequent procedures of bead loading, synthesis, and the on-array or off-array surface applications. Considerations for beads of choices are multiple: bead size can vary such as from um to nm; bead geometry and morphology may be spherical, hollow (nanoshell), square, elongated (rods); bead properties include magnetic, optical, or other types of active functions; bead materials may be polymers, crafted polymers, gold, silver, or other metallic composites, nanocystals, or other materials which can form functional beads in desirable dimensions. Beads of desirable properties possess one or a combination of the properties described above.

The method demonstrated herein are not limited to oligonucleotide synthesis and not limited to one type of molecule within one array site. It is well-appreciated by those spilled in art that peptides, oligonucleotide analogs, peptide analogs^{5 6}, or in general polyamide and polyphosphodiester sequences, carbohydrates³. Synthesis and medical applications of oligosaccharides. Nature 446, 1046-1051), and their conjugates. More than one type of molecules can be made on one array site using methods well known to those skilled in art such as randomization synthesis or orthogonal synthesis of two or more different molecules.

The method demonstrated herein provide a mixture of oligonucleotides with (one bead is loaded with one type of sequences) or without beads attached. The mixture of oligonucleotides have broad applications: the mixture bead samples can be used for target-specific applications such as probes for enrich or select DNA for sequencing for bead-based fast DNA sequencing such as the recent technologies from 454 Life Sciences and Roche (www.454.com) and Applied Biosystems (www;appliedsystems.com), genome-wide DNA sequencing from Solexa-illumina (www.illumina.com), nanoarrays, single molecule arrays, target specific realtime PCR, genome-wide amplification for various DNA analysis (SNP, Chip-on-Chip, CGH, methylation ampping), RNA sequencing and profiling, tags for purification, labeling, detection, or barcoding, bead arrays for assays of nucleic acids, proteins, and other types of molecules. These applications requires one, ten, 100, or 1,000 of the same molecules or the probes on beads and the bead-attached molecules target the same binding molecules, An array synthesizer producing fmol (6.0 x 10^8 molecules) will provide materials for 100,000 or more experiments, An array loaded with beads will provide materials for at least 1,000,000 or more experiments. Oligonucleotide mixtures also provides materials for synthetic DNA as described by Zhou et al (2004), which are precursors or building block materials for functional DNA constructs, RNAs, proteins, biopolymer complexes, minigenomes, organisms or cells.

In one embodiment of application, a set of 50,000 gene loci related to cancer gene activation (average length 1.5 kbp) for 1,000,000 test objects will be sequenced at 3x (three reads per sequence) (total 50,000,000,000 sequence read). This task can only be deal with by the current large scale sequencing technologies. However, the known technologies, such as 454 DNA sequencing using blank beads to randomly catch a sequence for sequencing. In this process, many beads catch too many sequences and many beads did not catch any sequence; the portion of the beads loaded with correct number of sequences follows typical statistical distribution. Furthermore, the beads loaded with a single sequence may be redundant, the tendency of which is depending on the abundance of a particular sequence; the higher the abundance, the high the chance that sequence will be populated on a larger number of beads. Therefore, there is a need for target-specific preparation of sequencing samples. A simple solution is to provide beads loaded with a single probe that can hybridize with a predetermined target. This simple solution, however, require high capacity oligonucleotide synthesis. A set of probes selecting the 3,000 genes from total RNA is provided so that at each sequencing run, the sequenced sequences are not by random choice but by hybridization selection. With this capability, the efficiency of each run is ensured and full sequence coverage is ensured or else, completion of the task is not possible. It is this type of deep sequencing effort that can help taking full advantage of the genome sequences available and gain insights that would not be possible if research dwells on low capacity low throughput, random experiments.

The present disclosure provides method for chemical modification on array surface of the molecules synthesized. The modifications introduce new properties to the array synthesized molecuies and thus open up new avenues for array applications which are beyond nucleic acid hybridization or typical peptide-based assays. One exemplary modification reaction is adding carbohydrate moiety to peptides through covalent linkages to create new molecules of modified peptides for screening antibody binding, cell receptor binding, protein binding, vaccine candidate screen, and other applications of normally involve carbohydrates and small. One useful reaction is Huisgen cycloaddition (click chemistry) (FIG. 15), which is to react a terminal alkyne (1505) with an azide (1510) compound to form a trizole (1515); this reaction conjugates the R and the R1 group to give an extended chain. The linker moiety in the azide compound 1520 may be an alkyl, ethylene glyco oligomer, polyamide, etc. Click chemistry has quickly gained popularity in last few years, due to the fact that reaction conditions are easy to accommodate and the compatibility with aqueous conditions suitable for biomolecular reactions. Described herein an experiment attaching an alpha-gal trisaccharide azide to peptides to form bioconjugates on an array surface. In a preferred example of the synthesis (FIG. 16), a peptide array (1605) contains Boc protected amino surface group. Selective deprotection of an array site to free surface amino group (1610) which in turn couples with propdic acid (X=nothing) (1615) to form a termus alkynyl (1620). Under click reaction conditions, the azide compound (1625) wherein R is a linker-linked fluoscein, the axido-dye (1625) is "clicked" to the terminal alkynyl to form the conjugate (Z=trizole) (1630). On this array surface the reaction steps of deprotection, coupling an alkynyl carboxylic acid, followed by Huisgen cyclioaddition are repeated, and a library of bioconjugates (1635), containing group R1, R2 or R3 at different array sites. In one example, the bioconjugates are peptide-derived compounds.

The modification of the molecules synthesized on an array is not limited to using click chemistry, other bioconjugation chemistry, such as cross-links formed by maleimide/thiol, aldehyde/amine or hydrazine, thiol/thiol, and the bis-aryl hydrazone bond (SoluLink. USA) are among the choices.

In one preferred example of the present disclosure, the reaction involved first synthesize the peptide sequences using photogenerated acids and Boc-chemistry as described previously. After the peptides were made, a first set of reaction sites were deprotected, and the amino groups were coupled with propiolic acid; a second set of sites were deprotected, and the amino groups were coupled with 4-pentynoic acid; the steps of deprotection and coupling were repeated to produce sites coupled with 6-heptynoic acid. These sites contain terminal alkyne groups. The peptide chip surface was then reacted with FITC-azide in ethanol and water mixture and in the presence of CuSO₄ and Ph₃P for 2 hours. A layout of the displayed image in FIG. 17 is provided at top. a fluorescence image of alkynyl terminated peptide sequences linked to FITC (fluorescein isothiocyanate) after the cycloaddition of FITC-azide and the surface alkynes. The displayed signals are consistent with the designed layout pattern, demonstrating the Huisgen cycloaddition works well on surface in array synthesis for modification of peptides.

In another preferred example of modification, the reaction conditions are similar to what described above and the selected deprotection step and coupling steps are arranged as such that the modifications are multiple at different positions of a sequence (1640) (FIG. 16).

The arrays containing modification molecules such as glycosylated peptides are of value as biomarker binding ligands due to their presentation of more functional groups compared to regular DNA or peptide molecules.

The present disclosure provides method of forming modified molecular arrays using in situ parallel synthesis methods based on activation of the synthesis cycles by direct photolithographic light irradiation, using photogenerated reagents of acid or base, electronically generated acid or base, or moving the reaction agent to the reaction site. The array of synthesis in not limited to certain sets of specific configurations, such as arrays of enclosed devices, or open surface, arrays in subsets or restricted areas, arrays of square centimeters or smaller sizes. However, it is critical that an array should allow modification reactions in a first predetermined area and then in a second predetermined area and so on.

The modifications and bioconjugation reactions on array surface are not limited to glycosyl conjugated peptides, oligonucteotides, carbohydrates, molecular moieties, these can be proteins, antibodies, cells, beads, such as TentaGel beads, Beads said herein also include colloidal CdSe-ZnS or other types of quantum dots found useful for tissue and in vivo staining, magnetic beads, coated magnetic beads found useful for quick purification of samples. Bead surface may be derivatized with functional groups such as hydroxyl OH, amino NH2. sulfuhhydryl SH, biotinyl, N-succinimidyl (NHS), azide, alkanyl, or alkynyl. Alternatively, bead surfaces may be coated with active molecules such as strepavidin, antibody, GST (glutathione S-transferase), HIS6 (HHHHHH), etc.

In a preferred embodiment of the present invention, one bead one compound of a total of thousands to millions of beads containing different compounds will be in great demand in next few years for high throughput assays and biochemical applications in a miniaturized format These beads may serve as probes for specific binding, substrates for enzymatic assays, primers for polymer chain extension, and numerous applications have been used daily now in a macroscale format One can envision to reduce the size of the current applications by using millions of beads of nm on a surface to form an array. This surface may or may not have grids to separate the beads. This surface may have coating to retain the beads. There are a large number of different types of beads made from different materials, such as gold, synthetic polymers and grafted synthetic polymers, sol-gel, and glass, and sizes ranging from nm to um.

The present disclosure provides method for preparing a bead library using array as a synthesis tool. Previous art of the well-known split-and-pool method produces a bead pool, but this method is laborious and the random arrays make difficult for well-controlled studies.. Robotic spotting or other methods of immobilizing molecules onto the beads have been used but these methods are only practical for a bead pool of thousands of different compounds. This is because the compounds are pre-synthesized and it is cost- and time-prohibitive to post-synthesize a larger number of compounds than a few thousands. The present disclosure provides method for creating a bead pool using array compounds synthesized on surface

In particular, method for preparation of a bead pool of directly "copy" the chemical content of a surface containing a variety of compounds is revealed A few examples of the disclosed method are given below

The synthesis of the array is performed as described in the publications⁵. For each of the biopolymers synthesized, an anchor moiety is incorporated, for instance at the terminus position Anchor molecules are those which can form covalent bond or high affinity bonding with incoming molecules After the array synthesis, beads to which the surface molecules can adhere, are loaded onto the surface The surface molecules will bind to the beads upon contacting The array surface molecules are then cleaved from the surface At each contact area, only one type of molecules is bound to the individual beads, and thus, the results of this experiment produce a collection of one-bead-one-compound in a bead pool

In another body experiment, the individual reaction sites are isolated after the beads distributed into the sites The binding of the surface molecules to the beads occurs and the surface molecules are cleaved from the surface The results of this experiment produce a collection of one-bead-one-compound in a bead pool.

In the experiment described, the individual reaction sites are isolated after the beads distributed into the sites. The binding of the surface molecules to the beads occurs after the surface molecules are cleaved from the surface The results of this experiment produce a collection of one-bead-one-compound in a bead pool

The molecules synthesized or immobilized on a surface at discrete sites For each of the biopolymers synthesized, an anchor moiety is incorporated for instance at the terminus position The anchor moiety is a reactive group or a group can be activated to form a linkage to the incoming beads After the synthesis, beads, to which the surface molecules can adhere are loaded onto the surface The surface molecules will bind to the beads upon contacting The surface molecules are then cleaved from the surface At each contact area, only one type of molecules is bound to the individual beads, and thus, the results of this experiment produces a collection of one-bead-one-compound in a bead pool

In the experiment described after the synthesis, beads to which the surface molecules can adhere are loaded onto the surface in a viscous media The surface molecules will bind to the beads upon contacting The surface molecules are then cleaved from the surface At each contact area, only one type of molecules is bound to the individual beads, and thus, the results of the experiment produces a collection of one-bead-one-compound in a bead pool

In the experiments described, the anchor moiety incorporated in the molecules synthesized may be a biotin moiety which can bind to streptavidin or avidin beads a thiol group which can bind to gold spheres, an amino group which can link to an aldehyde coated on beads, a thiol which reacts with maleimide on bead surface, or other type of ligand-host systems

The density of the surface molecules and diameters of the beads can be adjusted so that the number of the molecules attached to the bead can be controlled.

Tests showing the tight binding or bonding of the surface molecules with the beads causes the immobilization of the beads. The type of the anchor incorporated into the molecules synthesized on surface and the acceptor of the anchor moiety on the surface of the bead creates strong interactions so that the surface containing synthetic molecules is essentially "stricking" to the beads.

A preferred example of the present disclosure is the application of the bead library generated. Following the protocol described for "Ultrafast de novo sequencing of the human pathogen Corynebacterium urealyticum with the Genome Sequencer System" (www.454.com), a genomic DNA sample is obtained. Making an array of oligos containing the required sequence, the sequences are terminated with biotin, cleave the sequences from the array surface with 3'OH present Prepare a library of bead loaded oligos which are primers designed specific for the regions of DNA sequences of interest (DNA sequence information is available from NCBI or EBI information service sites); mix the beads with the genomic DNA, PCR to amplify the target DNA. This sample will be sequenced by the 454 Genome Sequencer. The target-specific sequencing will result in an increase in the total base reads and the coverage of the unique bases sequenced.

Various modifications and variation of the described method and system will be apparent to those skilled in the art. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology, genetics, chemistry or related fields are intended to be within the scope of the following claims.

### REFERENCES CITED

¹Lam, K S and Renil M (2002) From combinatorial chemistry to chemical microarray Current Opinion in Chemical Biology, 6 353-358
²Templin M. F Stoll, D. Schrenk, M , Traub, P C Vohringer, C F and Joos T O. (2002) Protein microarray technology Trends in Biotechnol 20 160 166
³Seeberger, P H Werz, D. B. (2007) Synthesis and medical applications of oligosaccharides Nature 446 1046-1051.
⁴Kuravilla, F G Shamji, A F Sternson, S M , Hergenother, P J and Schreiber S L (2002) Dissecting glucose signalling with diversity-oriented synthesis and small-molecule microarrays Nature 416 653-657
⁵Gao X Gulan, E and Zhou X (2004) in situ synthesis of oligonucleotide Microarrays Biopolymers 73,579-596
⁶Gao, X Pellois J P , Kim, K Na, Y Gulari. E , and Zhou X (2004) High density peptide microarrays. In situ synthesis and applications Molecular Diversity 8 177-187
⁷Pirrung, M. C., et al., Large scale photolithographic solid phase synthesis of pplypeptides and receptor binding Screening Thereof US5,143,8,54 1992-09-01.
⁸ Chee M. S., et al., Method of Making and Decoding of Array Sensors with Microspheres, US7060431 B2 200608-13.
⁹⁰ Singh-Gasson S etc., (1999) Maskless fabrication of light-directed of oligonucleotide microarrays using a digital micromirror array. Nature Biotechnology 17. 974 - 978
¹⁰Hughes, T R. et al (2001) Expression profiling using microarrays fabricated by an Ink-jet oligonucleotide synthesizer Nat Biotechnol. 19 342-347
¹¹Zhou, X Cal, S Hong, A., Yu. P Sheng N Srivannavit, O., Yong, Q., Muranjan, S Rouilard, J M Xia, Y., Zhang X , Xiang Q., Ganesh R., Zhu Q., Makejko, A Gulari, E., and Gao, X. (2004) Microfluidic picoarray synthesis of oligodeoxynucleotides and simultaneously assembling of multiple DNA sequences Nucleic Acids Res 32, 5409-5417.
¹²Gao X LeProust, E., Zhang H Srivannavit O., Gulari, E Yu, P., Nishiguchi. C., Xiang, Q., Zhou X. (2001) Flexible DNA chip Synthesis gated by deprotection using solution photogenerated acids. Nucleic Acids Res 29, 4744-4750.
¹³Peilois J. P., Zhou, X., Srivannavit, O., Zhou, T Gulari, E., and Gao X. (2002) Individually addressable parallel peptide synthesis on microchips Nat. Biotechnol. 20 922-926
¹⁴. Gao, X Pellois. J. J P., and Yao, W "Photogenerated reagents US6 965, 040; 2005-11-15 US7,235 670, 2007-06-26
¹⁵. Gao X., Zhou. X., and Gulari, E. "Method and apparatus for chemical and biochemical reactions using photo-generated reagents". US Patent 6 426,184 2002-07-30
¹⁶. Tian, J., Gong, H., Sheng, N Zhou X., Gulari. E., Gao, X and Church, G (2004) Accurate multiplex gene synthesis from programmable DNA chips Nature 432 1050-1054
¹⁷. Perez-Balderas. F., etc. (2003) Multivalent neoglycoconjugates by regiospecific cycloaddition of alkynes and azides using organic-soluble copper catalysts. Org Lett 5, 1951-1954.
¹⁸. Frank R (1992) SPOT-synthesis an easy technique for the positionally addressable, parallel chemical synthesis on a membrane support Tetrahedron 48, 9217-9232.
¹⁹. Flank, R (2002) The SPOT-synthesis technique Synthetic peptide arrays on membrane supports-principles and applications J Immunol Methods 267, 13-26.

## Claims

1. A solid surface comprising a density-variation molecular array having at least a first array site and a second array site wherein the first array site and the second array site comprise molecules synthesized in situ on respective first and second reaction sites having a predetermined density within each reaction site and where the predetermined density of molecules in the first reaction site is different from the predetermined density of molecules in the second reaction site; wherein the molecules in the first reaction site are present in a density different from that of the molecules in the second reaction site by at least approximately one thousand fold.

2. The solid surface of claim 1 wherein the molecules in the first reaction site are the same as in the second reaction site.

3. The solid surface of claim 1 wherein the molecules in the first reaction site are different as in the second reaction site.

4. The solid surface of claim 1 wherein the molecules in the first reaction site are present in a density different from that of the molecules in the second reaction site by at least approximately ten thousand fold.

5. The solid surface of claim 1 wherein the molecules in the first reaction site are present in a density different from that of the molecules in the second reaction site by at least approximately one hundred thousand fold.

6. The solid surface of claim 1 wherein the molecules in the first reaction site are present in a density different from that of the molecules in the second reaction site by at least approximately one million fold.

7. The solid surface of claim 1 further comprising a third array site, wherein the first array site, the second array site and third array site comprise molecules synthesized in situ on respective first, second and third reaction sites having a predetermined density within each site such that the predetermined density of molecules in the first reaction site is approximately 1000 fold greater than the predetermined density of molecules in the second array site and the predetermined density of molecules in the first array site is 10,000 fold greater than the predetermined density of molecules in the third array site.

8. The solid surface of claim 7 wherein the molecules in at least one of the reaction sites are different from the molecules in at least one of the other reaction sites.

9. The solid surface of claim 7 wherein the molecules in all of the reaction sites are the same.

10. The solid surface of claim 7 further comprising at least 1000 array sites.

11. The solid surface of claims 1 to 7 wherein the surface is divided into subsets such that each subset can be subjected to differential treatment during and after synthesis of the molecules.

## Patentansprüche

1. Feste Oberfläche, die eine Molekularanordnung mit Dichtevariation mit einer ersten Anordnungsstelle und einer zweiten Anordnungsstelle umfasst, wobei die erste Anordnungsstelle und die zweite Anordnungsstelle Moleküle umfassen, die in situ an entsprechenden ersten und zweiten Reaktionsstellen synthetisiert sind, mit einer vorbestimmten Dichte innerhalb jeder Reaktionsstelle, und wobei die vorbestimmte Dichte der Moleküle in der ersten Reaktionsstelle sich von der vorbestimmten Dichte der Moleküle in der zweiten Reaktionsstelle unterscheidet; wobei die Moleküle in der ersten Reaktionsstelle in einer Dichte vorhanden sind, die sich von der Dichte der Moleküle in der zweiten Reaktionsstelle um mindestens ungefähr das Tausendfache unterscheidet.

2. Feste Oberfläche nach Anspruch 1, wobei die Moleküle in der ersten Reaktionsstelle mit denen in der zweiten Reaktionsstelle übereinstimmen.

3. Feste Oberfläche nach Anspruch 1, wobei sich die Moleküle in der ersten Reaktionsstelle von denen in der zweiten Reaktionsstelle unterscheiden.

4. Feste Oberfläche nach Anspruch 1, wobei die Moleküle in der ersten Reaktionsstelle in einer Dichte vorhanden sind, die sich von der Dichte der Moleküle in der zweiten Reaktionsstelle um mindestens ungefähr das Zehntausendfache unterscheidet.

5. Feste Oberfläche nach Anspruch 1, wobei die Moleküle in der ersten Reaktionsstelle in einer Dichte vorhanden sind, die sich von der Dichte der Moleküle in der zweiten Reaktionsstelle um mindestens ungefähr das Hunderttausendfache unterscheidet.

6. Feste Oberfläche nach Anspruch 1, wobei die Moleküle in der ersten Reaktionsstelle in einer Dichte vorhanden sind, die sich von der Dichte der Moleküle in der zweiten Reaktionsstelle um mindestens ungefähr das Millionenfache unterscheidet.

7. Feste Oberfläche nach Anspruch 1, wobei diese ferner eine dritte Anordnungsstelle umfasst, wobei die erste Anordnungsstelle, die zweite Anordnungsstelle und die dritte Anordnungsstelle Moleküle umfassen, die in situ an entsprechenden ersten, zweiten und dritten Reaktionsstellen mit einer vorbestimmten Dichte in jeder Stelle synthetisiert sind, so dass die vorbestimmte Dichte der Moleküle in der ersten Reaktionsstelle ungefähr um das Tausendfache größer ist als die vorbestimmte Dichte der Moleküle in der zweiten Anordnungsstelle, und wobei die vorbestimmte Dichte der Moleküle in der ersten Anordnungsstelle um das Zehntausendfache größer ist als die vorbestimmte Dichte der Moleküle in der dritten Anordnungsstelle.

8. Feste Oberfläche nach Anspruch 7, wobei sich die Moleküle in mindestens einer der Reaktionsstellen von den Molekülen in mindestens einer der anderen Reaktionsstellen unterscheiden.

9. Feste Oberfläche nach Anspruch 7, wobei die Moleküle in allen Reaktionsstellen gleich sind.

10. Feste Oberfläche nach Anspruch 7, wobei diese ferner mindestens 1000 Anordnungsstellen umfasst.

11. Feste Oberfläche nach einem der Ansprüche 1 bis 7, wobei die Oberfläche in Teilgruppen unterteilt ist, so dass jede Teilgruppe während und nach der Synthese der Moleküle einer differentiellen Behandlung unterzogen werden kann.

## Revendications

1. Surface solide comprenant un réseau moléculaire à variation de densité ayant au moins un premier site de réseau et un deuxième site de réseau, dans laquelle le
premier site de réseau et le deuxième site de réseau comprennent des molécules synthétisées in situ sur des premier et deuxième sites de réaction respectifs ayant une densité prédéterminée à l'intérieur de chaque site de réaction et dans laquelle la densité prédéterminée de molécules dans le premier site de réaction est différente de la densité prédéterminée de molécules dans le deuxième site de réaction ; dans laquelle les
molécules dans le premier site de réaction sont présentes en une densité différente de celle des molécules dans le deuxième site de réaction d'au moins environ mille fois.

2. Surface solide selon la revendication 1, dans laquelle les molécules dans le premier site de réaction sont les mêmes que dans le deuxième site de réaction.

3. Surface solide selon la revendication 1, dans laquelle les molécules dans le premier site de réaction sont différentes de celles du deuxième site de réaction.

4. Surface solide selon la revendication 1, dans laquelle les molécules dans le premier site de réaction sont présentes en une densité différente de celle des molécules dans le deuxième site de réaction d'au moins approximativement dix mille fois.

5. La surface solide selon la revendication 1, dans laquelle les molécules dans le premier site de réaction sont présentes en une densité différente de celle des molécules
dans le deuxième site de réaction d'au moins environ cent mille fois.

6. La surface solide selon la revendication 1, dans laquelle les molécules dans le premier site de réaction sont présentes en une densité différente de celle des molécules dans le deuxième site de réaction d'au moins environ un million de fois.

7. Surface solide selon la revendication 1, comprenant en outre un troisième site de réseau, dans laquelle le premier site de réseau, le deuxième site de réseau et le troisième site de réseau comprennent des molécules synthétisées in situ sur les premier, deuxième et troisième sites de réaction respectifs ayant une densité prédéterminée dans chaque site de sorte que la densité prédéterminée de molécules dans le premier site de réaction est environ 1 000 fois supérieure à la densité prédéterminée de molécules dans le deuxième site de réseau et la densité prédéterminée de molécules dans le premier site de réseau est 10 000 fois supérieure à
la densité prédéterminée de molécules dans le troisième site de réseau.

8. Surface solide selon la revendication 7, dans laquelle les molécules dans au moins l'un des sites de réaction sont différentes des molécules dans au moins l'un des autres sites de réaction.

9. Surface solide selon la revendication 7, dans laquelle les molécules dans tous les sites de réaction sont les mêmes.

10. Surface solide selon la revendication 7, comprenant en outre au moins 1 000 sites de réseau.

11. Surface solide selon les revendications 1 à 7, dans laquelle la surface est divisée en sous-ensembles de sorte que chaque sous-ensemble peut être soumis à un traitement différent pendant et après la synthèse des molécules.
